# EUROPEAN PATENT APPLICATION

(11) **EP 4 144 857 A1**
(43) Date of publication of application: **08.03.2023**
(21) Application number: 20923578.7
(22) Date of filing: 13.07.2020
(51) Int. Cl.: C12N 15/873, C12N 15/11, C12N 15/06, A01K 67/027

(54) **ANIMAL PREPARATION METHOD**

(30) Priority: 06.03.2020 CN 202010151592; 30.04.2020 CN 202010375045
(71) Applicant: Mingceler Biotechnology Co., Ltd., Guangzhou, Guangdong 510525 (CN)
(72) Inventor: WU, Guangming, Guangzhou, Guangdong 510320 (CN); CHEN, Jiekai, Guangzhou, Guangdong 510320 (CN); WU, Kaixin, Guangzhou, Guangdong 510320 (CN)
(74) Representative: Grünecker Patent- und Rechtsanwälte PartG mbB
(86) International application number: PCT/CN2020/101681
(87) International publication number: WO 2021/174742

(57) **Abstract**

Provided is an animal preparation method and use thereof. The method includes aggregating a tetraploid embryo with embryonic stem cells to form a new reconstructed embryo or chimera embryo, the tetraploid embryo being a tetraploid embryo developed to 2-cell stage. By aggregating a 2-cell tetraploid embryo with embryonic stem cells, problems of poor efficiency and poor stability of mice preparation using the tetraploid complementation technique as well as low efficiency when embryonic stem cells from pure line mice are used are alleviated, the birth rate of mice is improved to a level close to that of normal embryo transplantation, and embryos and adult mice can be directly prepared from stem cells for phenotypic research.

## Description

### Field of the Invention

The present disclosure belongs to the field of biomedicines, and relates to an animal preparation method.

### Background of the Invention

Laboratory animal models of diseases are indispensable research tool for studying the etiology and pathogenesis of human diseases, and for developing prevention and treatment technologies and therapeutic drugs. Common laboratory animals include mice, rats, guinea pigs, hamsters, rabbits, dogs, monkeys, pigs and fishes. However, there are plenty of differences in gene and protein sequences between human and animals. Many human proteins cannot bind to homologous proteins in animals to produce biological activity, leading to problems that many clinical test results are inconsistent with the results from animal experiments.

With the continuous development and maturity of genetic engineering technology, human cells or genes can be used to substitute or replace corresponding endogenous cells or genes in animals, to establish biological systems or disease models (e.g. humanized animal models) that are closer to humans. This has provided an important tool for new clinical therapeutic methods or means. For the gene humanized animal model, a corresponding gene of an animal is replaced with a wild-type or mutant gene of human using genetic engineering technology. In this way, it can establish, in the animal body, the wild-type or mutant gene closer to human. Due to the existence of human gene fragments, a full-length or part of a human protein with functions can be expressed in animal bodies, so as to greatly reduce the differences in the results between human and animal clinical experiments. Thus, it is possible to conduct drug screening at the animal level.

Both 2019-nCoV and SARS coronaviruses use angiotensin-converting enzyme 2 (ACE2) as a key target for infecting humans. Among them, 2019-nCoV, in addition to infecting humans, can also infect a variety of mammals such as monkeys, pigs, rabbits, ferrets, orangutans, but cannot infect mice and rats. As animal models infecting with 2019-nCoV for drug screening, monkeys, pigs and rabbits are difficult to be processed in large quantities due to their long growth cycles and large body sizes. In contrast, mice can be relatively easy to be processed in large quantities. However, mice are insusceptible to 2019-nCoV.

Tetraploid means that a cell contains four times the number of monoploid chromosomes. That is, the tetraploid is an individual or a cell having four complete sets of chromosomes. Under natural conditions, mammalian tetraploid embryos are infrequent and generally cannot normally develop into a single individual.

When a certain number of embryonic stem (ES) cells and a tetraploid embryo are chimerized, during the development of the chimera, the ES cells and the tetraploid embryo are not randomly distributed. The tetraploid embryo only participates in the formation of extraembryonic tissues such as yolk sac endoderm and placental trophoblast cells (e.g., chorionic ectoderm, trophoblast cells, etc.). The ES cells are extensively involved in the generation of embryoid body, allantois, amniotic membrane, yolk sac mesoderm and chorionic mesoderm, but not involved in the generation of yolk sac endoderm and placental trophoblast cell lineage. In other words, the developmental potencies of the ES cells and the tetraploid embryo are complementary. This phenomenon is called tetraploid complementation.

Animals cloned by this technology are completely developed from ES cells, and are called ES animals. The cloned embryos prepared by nuclear transplantation, when implanted to surrogate mothers, result in an extremely low fetal birth rate, only 1-2%. In contrast, the tetraploid complementation technique, by which an ES cell line is injected into a tetraploid embryo vector at the blastocyst stage, can increase the fetal birth rate to 20-30%, and significantly improve the efficiency of animal cloning. In recent years, many scientists in China and abroad have conducted in-depth research on this technique and have produced many new research findings. Animal models generated by the tetraploid complementation technique have a wide range of applications, such as in determining lineage-specific gene functions, analyzing gene functions in extraembryonic and embryonic tissues, identifying cell-spontaneous and non-spontaneous gene functions, distinguishing primary defects from secondary defects, facilitating phenotypic analysis and the like. The application of embryo chimeras is not limited to the study of early embryonic development, but can also be expanded to the study of development and body functions of fetuses after birth. When the chimeras are used in conjunction with molecular means, such molecular means can modify genetic activity in cells in a time- and lineage-specific manner, thereby facilitating precise, in-depth, and large-scale analysis of gene functions. Genetic activity can be altered (Nagy 2000) or gene function can be modulated by conditionally altering gene expression. At present, the evaluation of the developmental potency of ES cells mainly focuses on the study of cell differentiation in vitro and the analysis of transcriptional activity of molecular markers reflecting cell pluripotency. A unique feature of embryonic chimeras is that foreign cells can be chimerized into an embryo, and the cells can fully stimulate the differentiation potential during embryonic development. The cells are tested for comprehensive cell lineages, which can reveal the true extent of pluripotency more efficiently. The chimeras have become the most comprehensive and rigorous *in vivo* evaluation means for predicting mammalian pluripotent cells.

### Summary of the Invention

In some embodiments, the present disclosure provides a preparation method capable of increasing the birth rate of an animal.

In some embodiments, the present disclosure provides an animal tetraploid embryo complementation assay.

In some embodiments, the present disclosure provides a method for preparing an animal. The method includes aggregating a tetraploid embryo with embryonic stem cells to form a new reconstructed embryo or a chimeric embryo, wherein the tetraploid embryo is a tetraploid embryo at 2-cell stage

In some embodiments, the embryonic stem cells are embryonic stem cells within passage 15 (p15).

In some embodiments, the embryonic stem cells are embryonic stem cells at p12-p15.

In some embodiments, the method uses a tetraploid complementation assay or a tetraploid embryo complementation assay.

In some embodiments, the method comprises the following steps: (1) obtaining an animal 2-cell embryo; (2) placing the 2-cell embryo obtained in step (1) in a fusion solution and performing fusion to obtain a tetraploid embryo; (3) placing the tetraploid embryo obtained in step (2) in a culture medium and culturing; (4) aggregating the tetraploid embryo, which is developed to 2-cell stage in step (3), with embryonic stem cells to form a chimeric embryo; and (5) implanting the chimeric embryo obtained in step (4) into a uterus of a pseudopregnant animal to develop the chimeric embryo to full term to obtain the animal.

In some embodiments, in step (3), the tetraploid embryo is cultured for 8-24 hours.

In some embodiments, the culture medium in step (3) is KSOM medium.

In some embodiments, the non-human animal derived from the recipient's embryo may be any animal other than humans, such as a pig, a rat, a mouse, a hamster, a rabbit, a pig, a bovine, a deer, a sheep, a goat, a chicken, a cat, a horse, a dog, an orangutan, and a monkey. Meanwhile, the mammal used as the original source of the cells to be transplanted into the recipient may be a human or a mammal other than humans, for example, a pig, a rat, a mouse, a bovine, a sheep, a goat, a horse, a dog, a baboon, a chimpanzee, a gorilla, an orangutans, a monkey, a marmoset, a bonobo and the like.

In some embodiments, the animal is a mammal.

In some embodiments, the animal is a non-human mammal.

In some embodiments, the animal is selected from a group consisting of pig, rat, mouse, hamster, rabbit, pig, bovine, deer, sheep, goat, chicken, cat, horse, dog, orangutan, and monkey.

In some embodiments, the animal is selected from a murine. In some embodiments, the animal is selected from an adult murine. In some embodiments, the animal is selected from a fetal murine.

Existing technologies show that current technologies for preparing animals are not able to meet the requirements of practical application. For example, in the preparation of mice, early studies have found that mouse tetraploid embryos cannot develop normally and can only form trophoblast and extraembryonic endoderm of a placenta (Tarkowski et al., 1977), while embryonic stem cells can form an entire fetus as well as yolk sac mesoderm, amniotic membrane, allantois, umbilical cord, etc., but have very limited ability to form trophoblast and yolk sac endoderm (Beddington et al., 1989). The tetraploid complementation technique was developed right based on this perfect complementary relationship. In the most extreme cases, the use of embryonic stem cells with the best pure-line genetic background could result in a fetal birth rate of up to 15%, but these mice all died shortly after birth (Nagy et al., 1990).

It was later found that chimeric embryos obtained using embryonic stem cells with F1 genetic background at an early passage could develop to full term and live to adulthood, but the efficiency was only 3.8% (Nagy et al., 1993). It was later reported that the survival rate of F1 embryonic stem cells, after gene targeting and recloning, could reach 5-15%, but the survival rate of the mice produced using pure-line embryonic stem cells was still only 0-1.4% (Eggan et al., 2001). In 2006, Guy S Eakin et al. disclosed a method of preparing mice by aggregating embryonic stem cells with a diploid or a tetraploid (Eakin and Hadjantonakis, 2006). Other methods are also disclosed (Gertsenstein, 2015; Kang and Gao, 2015; Li et al., 2015; Shinozawa et al., 2006; Tanaka et al., 2009; Wen et al., 2014; Yamaguchi et al., 2018; Zhao et al., 2009; Zhao et al., 2010). In 2009, Michal J. Boland et al. prepared mice from mouse induced pluripotent stem cells using the tetraploid complementation technique, with the birth rate of mice being 0-7%, mostly 0% (Boland et al., 2009). The birth rate published by Zhou Qi in the international journal NATURE was merely between 0-3% (Zhao et al., 2009). Although the efficiency of producing mice using embryonic stem cells with F1 background by the tetraploid complementation technique is increased by way of large-scale cell line screening, technical improvements, and recloning, F1 is not an ideal genetic background, and this technique is still unable to meet requirements of practical applications. Production of transgenic mice with pure-line embryonic stem cells by tetraploid complementation technique, in particular, still faces great difficulties.

In some embodiments, the present disclosure provides a method for preparing a gene-edited animal using the animal preparation method. In some embodiments, the animal is an animal with a humanized gene. In some embodiments, the animal is a mammal. In some embodiments, the animal is a non-human mammal. In some embodiments, the animal is selected from a group consisting of pig, rat, mouse, hamster, rabbit, pig, bovine, deer, sheep, goat, chicken, cat, horse, dog, orangutan, and monkey. In some embodiments, the rodent is a murine. In some embodiments, the animal is an adult murine. In some embodiments, the animal is a fetal murine.

A gene-edited mouse is usually obtained by the following ways: blastocyst injection of pluripotent stem cells, direct editing by zygote injection, somatic cell nuclear transfer, tetraploid complementation technique, etc. Blastocyst injection of pluripotent stem cells requires that germline-transmitted chimeric mice be obtained and then subjected to cross-generation reproduction to obtain homozygous mice, which takes more than 5 months. Zygote injection is usually used for gene knockout editing, and is limited in gene editing strategies and difficult to operate; it also requires subsequent identification of born mice and may not necessarily lead to direct obtaining of target mice. Both somatic cell nuclear transfer and tetraploid complementation technique can be used to directly obtain homozygous gene-edited mice, but the two methods both result in a very low survival rate of less than 10%, usually around 1%. In addition, somatic cell nuclear transfer is difficult to operate. Microinjection can cause great damage to embryos, while tetraploid complementation technique will not cause direct damage to embryos. For direct obtaining of gene-edited mice, tetraploid complementation technique is simpler and more efficient than somatic cell nuclear transfer, takes a shorter period time to obtain gene-edited animals, and is relatively easy to perform gene editing on pluripotent stem cells.

In some embodiments, a method for preparing a genetic mouse is provided. The method includes the following steps: (1) obtaining a mouse 2-cell embryo; (2) placing and fusing the 2-cell embryo obtained in step (1) in a fusion solution to obtain a tetraploid embryo; (3) placing and culturing the tetraploid embryo obtained in step (2) in a culture medium; (4) aggregating the tetraploid embryo developed to 2-cell stage in step (3) with embryonic stem cells to form a chimeric embryo; (5) implanting the chimeric embryo obtained in step (4) into a uterus of a pseudopregnant mouse to develop the chimeric embryo to full term so as to obtain a gene-edited mouse.

In some embodiments, the method includes aggregating embryonic stem cells with a tetraploid embryo to form a new reconstituted embryo. The embryonic stem cells may be ordinary embryonic stem cells, or gene-edited embryonic stem cells, such as animal embryonic stem cells with a humanized gene, which are not particularly limited.

In some embodiments, a method for preparing an animal chimeric embryo is provided. The method includes aggregating a tetraploid embryo with embryonic stem cells to form a new reconstructed embryo or a chimeric embryo, wherein the tetraploid embryo is a tetraploid embryo at 2-cell stage.

In some embodiments, the method adopts a tetraploid complementation assay or a tetraploid embryo complementation assay.

In some embodiments, the method includes the following steps: (1) obtaining an animal 2-cell embryo; (2) placing the 2-cell embryo obtained in step (1) in a fusion solution and performing fusion to obtain a tetraploid embryo; (3) placing the tetraploid embryo obtained in step (2) in a culture medium and culturing; and (4) aggregating the tetraploid embryo, which is developed to 2-cell stage in step (3), with embryonic stem cells to form a chimeric embryo. In some embodiments, the tetraploid embryo is cultured for 8-24 hours. In some embodiments, the culture medium in step (3) is KSOM medium. In some embodiments, the animal is a mammal. In some embodiments, the animal is a non-human mammal. In some embodiments, the animal is selected from a group consisting of pig, rat, mouse, hamster, rabbit, pig, bovine, deer, sheep, goat, chicken, cat, horse, dog, orangutan, and monkey. In some embodiments, the animal is selected from a murine. In some embodiments, the animal is selected from an adult murine. In some embodiments, the animal is selected from a fetal murine.

In some embodiments, the method includes aggregating a tetraploid embryo developed to 2-cell stage with embryonic stem cells to obtain a chimeric embryo. The inventors unexpectedly found that, by aggregating a 2-cell tetraploid embryo with embryonic stem cells, problems of poor efficiency and poor stability of mice preparation using the tetraploid complementation technique as well as low efficiency when embryonic stem cells from pure line mice are used are alleviated, and the birth rate of mice is significantly improved to a level close to that of normal embryo transplantation. At the same time, it overcomes limitations of the currently most advanced gene editing method Cas9, and makes it possible to directly produce embryos and adult mice from stem cells for phenotype research. This helps to save a lot of time, manpower and material resources required in mice reproduction, and thoroughly solves practical problems of tetraploid complementation technique in preparing transgenic mice, reaching a commercialized level.

In some embodiments, the method for preparing an animal uses a composition comprising mannitol, MgSO₄, CaCl₂ and bovine serum albumin in a mass ratio of 9-53 : 0.015-0.241 : 0.013-0.23 : 0.01-5.

In some embodiments, the method uses a composition comprising mannitol, MgSO₄, CaCl₂ and bovine serum albumin in a mass ratio of 9-53 : 0.015-0.241 : 0.013-0.23 : 0.01-5.

In some embodiments, the composition comprises mannitol, MgSO₄, CaCl₂ and bovine serum albumin in a mass ratio of 18-52 : 0.018-0.241 : 0.016-0.23 : 0.01-4.

In some embodiments, the composition comprises mannitol, MgSO₄, CaCl₂ and bovine serum albumin in a mass ratio of 27-52 : 0.018-0.12 : 0.016-0.1 : 1-3.5.

In some embodiments, the composition comprises 0.05-0.29 M of mannitol, 0.12-2 mM of MgSO₄, 0.12-2 mM of CaCl₂, and 0.01-5 mg/mL of bovine serum albumin.

In some embodiments, the composition comprises 0.1-0.28 M of mannitol, 0.15-2 mM of MgSO₄, 0.15-2 mM of CaCl₂, and 0.01-4 mg/mL of bovine serum albumin.

In some embodiments, the composition comprises 0.15-0.28 M of mannitol, 0.15-1 mM of MgSO₄, 0.15-1 mM of CaCl₂, and 1-3.5 mg/mL of bovine serum albumin.

In some embodiments, the composition comprises 0.18-0.28 M of mannitol, 0.15-0.5 mM of MgSO₄, 0.15-0.5 mM of CaCl₂, and 2-3.5 mg/mL of bovine serum albumin.

In some embodiments, the composition comprises 0.2-0.28 M of mannitol, 0.15-0.3 mM of MgSO₄, 0.15-0.3 mM of CaCl₂, and 2-3 mg/mL of bovine serum albumin

In some embodiments, the composition comprises 0.27 M of mannitol, 0.2 mM of MgSO₄, 0.2 mM of CaCl₂, and 3 mg/mL of bovine serum albumin.

In some embodiments, the composition may be used as a stock solution and then formulated into a working solution of a desired concentration.

In some embodiments, the composition may be a working solution that can be used directly without formulation.

In some embodiments, the present disclosure provides use of the composition in preparation of gene-edited animal embryonic cells or gene-edited animals.

In some embodiments, the composition is used to prepare animal tetraploid embryos.

In some embodiments, the method uses a fusion solution containing mannitol, bovine serum albumin, Mg²⁺ and Ca²⁺, the fusion solution comprises increased concentrations of Mg²⁺ and Ca²⁺; alternatively, the fusion solution comprises 0.12-2 mM of Mg²⁺ and 0.12-2 mM of Ca²⁺.

In some embodiments, the fusion solution comprises 0.05-0.29M of mannitol and 0.01-5 mg/mL of bovine serum albumin.

In some embodiments, the Mg²⁺ and Ca²⁺ are derived from MgSO₄ and CaCl₂, respectively.

In some embodiments, the fusion solution is used for preparing the tetraploid embryo.

At present, methods of obtaining tetraploids include biological, chemical and physical methods. Injection of two diploid embryonic nuclei into the cytoplasm of a 1-cell embryo by micromanipulation requires high operational skills, and micromanipulation can cause great damage to the embryo. Preparation of a tetraploid by using cytochalasin B or colchicine to inhibit the division of blastomere cells can have adverse effects on embryonic development, and even delay embryonic development. Methods such as Sendai virus, polyethylene glycol, and direct current electric shock can also be used for cell fusion. Sendai virus is pathogenic, and polyethylene glycol has residual toxicity. The electrofusion method, however, only produces reversible electroporation in a short period of time and avoids the toxicity of chemical reagents and the pathogenicity of viruses, and is thus the most commonly used and efficient method for preparing tetraploid embryos. Whereas the existing tetraploid complementation technique still has the problems that the electrofusion efficiency of embryos is less than 100%, the efficiency and stability in mice preparation are poor, and the efficiency when using embryonic stem cells from pure mice is low. During research, the inventors unexpectedly found that the improved electrofusion solution of the present disclosure can increase the fusion efficiency from 80-90% to 100%, which has great practical significance.

In some embodiments, the present disclosure provides an animal prepared by the method.

In some embodiments, the animal is a mammal. In some embodiments, the animal is a non-human mammal. In some embodiments, the animal is selected from a group consisting of pig, rat, mouse, hamster, rabbit, pig, bovine, deer, sheep, goat, chicken, cat, horse, dog, orangutan, and monkey. In some embodiments, the animal is selected from a murine. In some embodiments, the animal is selected from an adult murine. In some embodiments, the animal is selected from a fetal murine. In some embodiments, the animal is selected from gene-edited animals. In some embodiments, the animal is selected from an animal with a humanized gene. In some embodiments, the gene is ACE2.

In some embodiments, the present disclosure provides a fusion solution containing mannitol, bovine serum albumin, Mg²⁺ and Ca²⁺, the fusion solution comprises increased concentrations of Mg²⁺ and Ca²⁺; alternatively, the fusion solution comprises 0.12-2 mM of Mg²⁺ and 0.12-2 mM of Ca²⁺.

In some embodiments, the fusion solution comprises 0.05-0.29 M of mannitol and 0.01-5 mg/mL of bovine serum albumin.

In some embodiments, the Mg²⁺ and Ca²⁺ are derived from MgSO₄ and CaCl₂, respectively.

In some embodiments, the method for preparing an animal uses the fusion solution.

In some embodiments, the present disclosure provides tissues, body fluids, cells, nuclei, and debris or extracts thereof, of a gene-edited animal or its offspring constructed using the above method. In some embodiments, the animal is a mammal. In some embodiments, the animal is a non-human mammal. In some embodiments, the animal is selected from a group consisting of pig, rat, mouse, hamster, rabbit, pig, bovine, deer, sheep, goat, chicken, cat, horse, dog, orangutan, and monkey. In some embodiments, the animal is selected from a murine. In some embodiments, the animal is selected from an adult murine. In some embodiments, the animal is selected from a fetal murine.

Due to differences in gene sequences between human ACE2 and mouse ACE2, it has been reported in the literature that after virus infection, human ACE2 is more sensitive to SARS-CoV than mouse ACE2, and leads to more obvious pathological symptoms. Better animal models are urgently needed for current clinical studies.

There are hACE2 transgenic mice having been confirmed to be able to be infected with 2019-nCoV and develop typical pathogenic characteristics in lung tissues (https://www.biorxiv.org/content/10.1101/2020.02.07.939389v3#disqus thread). However, hACE2 transgenic mice are mouse models with systemic overexpression of hACE2, and cannot mimic spatiotemporal expression characteristics of ACE2.

In some embodiments, the present disclosure will mass produce 2019-nCoV-susceptible mouse humanized ACE2 animal models by expressing humanized ACE2 at mouse ACE2 sites to do drug screening and disease research.

In some embodiments, the present disclosure provides a specific RNA fragment sequence.

In some embodiments, the RNA fragment sequence is an isolated RNA fragment sequence.

In some embodiments, the present disclosure provides a specific sgRNA sequence targeting ACE2 gene.

In some embodiments, the sgRNA sequence is an isolated sgRNA sequence. In some embodiments, the present disclosure provides a specific targeting vector targeting ACE2 gene.

In some embodiments, the targeting vector is an isolated targeting vector.

In some embodiments, the present disclosure provides an ACE2 gene humanized cell strain.

In some embodiments, the present disclosure provides a method for preparing an ACE2 gene humanized cell strain.

In some embodiments, the present disclosure provides an ACE2 gene humanized animal.

In some embodiments, the present disclosure provides a method for preparing an animal with a humanized gene.

In some embodiments, the present disclosure provides an ACE2 gene humanized mouse embryonic stem cell model.

In some embodiments, the present disclosure provides a set of primers comprising an upstream primer that has at least 85% or at least 90% or at least 91% or at least 92% or at least 93% or at least 94% or at least 95% or at least 96% or at least 97% or at least 98% or at least 99%, or 100% identity to a sequence as shown by SEQ ID NO: 19; and a downstream primer that has at least 85% or at least 90% or at least 91% or at least 92% or at least 93% or at least 94% or at least 95% or at least 96% or at least 97% or at least 98% or at least 99%, or 100% identity to a sequence as shown by SEQ ID NO: 20.

In some embodiments, the above primers are separate primers.

In some embodiments, the set of primers further comprises an upstream primer that has at least 85% or at least 90% or at least 91% or at least 92% or at least 93% or at least 94% or at least 95% or at least 96% or at least 97% or at least 98% or at least 99%, or 100% identity to a sequence as shown by SEQ ID NO: 21 and a downstream primer that has at least 85% or at least 90% or at least 91% or at least 92% or at least 93% or at least 94% or at least 95% or at least 96% or at least 97% or at least 98% or at least 99%, or 100% identity to a sequence as shown by SEQ ID NO: 22.

In some embodiments, the above primers are separate primers.

In some embodiments, the set of primers further comprises an upstream primer that has at least 85% or at least 90% or at least 91% or at least 92% or at least 93% or at least 94% or at least 95% or at least 96% or at least 97% or at least 98% or at least 99%, or 100% identity to a sequence as shown by SEQ ID NO: 23, and a downstream primer that has at least 85% or at least 90% or at least 91% or at least 92% or at least 93% or at least 94% or at least 95% or at least 96% or at least 97% or at least 98% or at least 99%, or 100% identity to a sequence as shown by SEQ ID NO:24.

In some embodiments, the above primers are separate primers.

In some embodiments, the set of primers further comprises an upstream primer that has at least 85% or at least 90% or at least 91% or at least 92% or at least 93% or at least 94% or at least 95% or at least 96% or at least 97% or at least 98% or at least 99%, or 100% identity to a sequence as shown by SEQ ID NO: 25 and a downstream primer that has at least 85% or at least 90% or at least 91% or at least 92% or at least 93% or at least 94% or at least 95% or at least 96% or at least 97% or at least 98% or at least 99%, or 100% identity to a sequence as shown by SEQ ID NO: 26.

In some embodiments, the above primers are separate primers.

In some embodiments, the present disclosure provides use of the set of primers in preparation of a cell strain or a cell line or a non-human animal.

In some embodiments, the cell above is an isolated cell.

In some embodiments, the animal is selected from a group consisting of pig, rat, mouse, hamster, rabbit, pig, bovine, deer, sheep, goat, chicken, cat, horse, dog, orangutan, and monkey. In some embodiments, the mammal is a rodent animal. In some embodiments, the animal is a murine. In some embodiments, the animal is selected from an adult murine. In some embodiments, the animal is selected from a fetal murine.

In some embodiments, the animal is an animal with a humanized gene.

In some embodiments, the gene is ACE2.

In some embodiments, the present disclosure provides use of the set of primers in preparation of a targeting vector.

In some embodiments, the present disclosure provides a targeting vector, comprising a 5' homology arm, a fragment of human ACE2 gene, and a SV40 polyA that are linked. The 5' homology arm is homologous to a 5' target sequence of a target locus in a genome.

In some embodiments, the targeting vector described above is an isolated targeting vector.

In some embodiments, the 5' homology arm, the fragment of human ACE2 gene, and the SV40 polyA sequence are linked and inserted into the targeting vector using the set of primers.

In some embodiments, the targeting vector is used for inserting a CDS region of human ACE2 gene downstream of a promoter and 5' UTR region of an animal gene, so as to initiate expression of the human gene of interest using the promoter of the animal gene.

In some embodiments, the 5' homology arm comprises a sequence having at least 85% or at least 90% or at least 91% or at least 92% or at least 93% or at least 94% or at least 95% or at least 96% or at least 97% or at least 98% or at least 99%, or 100% identity to the sequence as shown by SEQ ID NO: 15.

In some embodiments, the CDS region of ACE2 gene comprises a sequence having at least 85% or at least 90% or at least 91% or at least 92% or at least 93% or at least 94% or at least 95% or at least 96% or at least 97% or at least 98% or at least 99%, or 100% identity to the sequence as shown by SEQ ID NO: 12.

In some embodiments, the SV40 polyA comprises a sequence having at least 85% or at least 90% or at least 91% or at least 92% or at least 93% or at least 94% or at least 95% or at least 96% or at least 97% or at least 98% or at least 99%, or 100% identity to the sequence as shown by SEQ ID NO: 14.

In some embodiments, the SV40 polyA is located downstream of the CDS region.

In some embodiments, the targeting vector further comprises a 3' homology arm which is homologous to a 3' target sequence of the target locus in the genome.

In some embodiments, the targeting vector further comprises a selection marker PGK-Puro that has a sequence having at least 85% or at least 90% or at least 91% or at least 92% or at least 93% or at least 94% or at least 95% or at least 96% or at least 97% or at least 98% or at least 99%, or 100% identity to the sequence as shown by SEQ ID NO: 17.

In some embodiments, the targeting vector further comprises a FLP Recombination Target (Frt) site that has a sequence having at least 85% or at least 90% or at least 91% or at least 92% or at least 93% or at least 94% or at least 95% or at least 96% or at least 97% or at least 98% or at least 99%, or 100% identity to the sequence as shown by SEQ ID NO: 18.

In some embodiments, the targeting vector comprises, linked in turn: the 5' homology arm, the fragment of human ACE2 gene, the SV40 polyA, the Frt site, the PGK-Puro, the Frt site, and the 3' homology arm.

In some embodiments, the animal is a mammal. In some embodiments, the animal is selected from a group consisting of pig, rat, mouse, hamster, rabbit, pig, bovine, deer, sheep, goat, chicken, cat, horse, dog, orangutan, and monkey. In some embodiments, the mammal is a rodent animal. In some embodiments, the animal is a murine. In some embodiments, the animal is selected from an adult murine. In some embodiments, the animal is selected from a fetal murine.

In some embodiments, the animal is an animal with a humanized gene.

In some embodiments, the gene is ACE2.

In some embodiments, the present disclosure provides use of the targeting vector in preparation of an animal model which has a humanized gene. The humanized ACE2 mouse model is a mouse model that specifically expresses hACE2 at original mouse ACE2 sites, and can simulate the expression characteristics of the original mACE2, has tissue specificity, and can more rigorously simulates the pathogenesis of human infection with 2019-nCoV.

In some embodiments, the animal is a mammal. In some embodiments, the animal is selected from a group consisting of pig, rat, mouse, hamster, rabbit, pig, bovine, deer, sheep, goat, chicken, cat, horse, dog, orangutan, and monkey. In some embodiments, the mammal is a rodent animal. In some embodiments, the animal is a murine. In some embodiments, the animal is selected from an adult murine. In some embodiments, the animal is selected from a fetal murine.

In some embodiments, the animal is an animal with a humanized gene.

In some embodiments, the gene is ACE2.

In some embodiments, the present disclosure provides a method for preparing the targeting vector. The method includes the following step: performing an overlap PCR reaction using PCR amplification products of the 5' homology arm, the CDS region of human ACE2 gene, and the SV40 poly A, to obtain a continuous fragment 5arm-hACE-SV40.

In some embodiments, a system of the overlap PCR reaction comprises: about 25 µL of 2×Phanta Max Buffer; about 1 µL of dNTP Mix; about 2 µL of 10 µM upstream primer; about 2 µL of 10 µM downstream primer; about 1 µL of DNA Polymerase; about 20-200 ng of each of the PCR amplification products of the 5' homology arm, the CDS region of human ACE2 gene, and SV40 polyA as template; and H₂O, making up to about 50 µL. The overlap PCR reaction comprises the steps of: starting the PCR reaction at about 62-68°C, and decreasing t by about 0.2-0.5°C each cycle.

In some embodiments, a set of primers for amplifying the 5' homology arm comprises an upstream primer that has a sequence having at least 85% or at least 90% or at least 91% or at least 92% or at least 93% or at least 94% or at least 95% or at least 96% or at least 97% or at least 98% or at least 99%, or 100% identity to the sequence as shown by SEQ ID NO: 19 and a downstream primer that has a sequence having at least 85% or at least 90% or at least 91% or at least 92% or at least 93% or at least 94% or at least 95% or at least 96% or at least 97% or at least 98% or at least 99%, or 100% identity to the sequence as shown by SEQ ID NO: 20.

In some embodiments, the primers are isolated primers.

In some embodiments, a set of primers for amplifying the CDS region of human ACE2 gene comprises an upstream primer that has a sequence having at least 85% or at least 90% or at least 91% or at least 92% or at least 93% or at least 94% or at least 95% or at least 96% or at least 97% or at least 98% or at least 99%, or 100% identity to the sequence as shown by SEQ ID NO: 21 and a downstream primer that has a sequence having at least 85% or at least 90% or at least 91% or at least 92% or at least 93% or at least 94% or at least 95% or at least 96% or at least 97% or at least 98% or at least 99%, or 100% identity to the sequence as shown by SEQ ID NO: 22.

In some embodiments, the primers are isolated primers.

In some embodiments, a set of primers for amplifying the SV40 polyA comprises an upstream primer that has a sequence having at least 85% or at least 90% or at least 91% or at least 92% or at least 93% or at least 94% or at least 95% or at least 96% or at least 97% or at least 98% or at least 99%, or 100% identity to the sequence as shown by SEQ ID NO: 23 and a downstream primer that has a sequence having at least 85% or at least 90% or at least 91% or at least 92% or at least 93% or at least 94% or at least 95% or at least 96% or at least 97% or at least 98% or at least 99%, or 100% identity to the sequence as shown by SEQ ID NO: 24.

In some embodiments, the primers are isolated primers.

In some embodiments, the method further comprises the following steps: subjecting the fragment 5arm-hACE-SV40 to AgeI+MluI double digestion, subjecting the 3' homology arm to AscI+HindIII double digestion, and ligating the digested fragments respectively, so as to obtain the targeting vector.

In some embodiments, a set of primers for the 3' homology arm fragment comprises an upstream primer that has a sequence having at least 85% or at least 90% or at least 91% or at least 92% or at least 93% or at least 94% or at least 95% or at least 96% or at least 97% or at least 98% or at least 99%, or 100% identity to the sequence as shown by SEQ ID NO: 25 and a downstream primer that has a sequence having at least 85% or at least 90% or at least 91% or at least 92% or at least 93% or at least 94% or at least 95% or at least 96% or at least 97% or at least 98% or at least 99%, or 100% identity to the sequence as shown by SEQ ID NO: 26.

In some embodiments, the primers are isolated primers.

In some embodiments, the present disclosure provides a method for preparing a humanized animal cell strain using the set of primers.

In some embodiments, the method includes a step of using the targeting vector.

In some embodiments, the method includes a step of introducing a target human-derived gene into an animal cell, so that the animal cell has CDS of the target human-derived gene.

In some embodiments, the target gene is ACE2.

In some embodiments, the animal is a mammal. In some embodiments, the animal is a rodent animal. In some embodiments, the animal is a murine.

In some embodiments, the cell is an embryonic stem cell.

In some embodiments, the preparation method includes the following steps: (1) preparing the targeting vector; (2) introducing the prepared targeting vector and a vector linked with an sgRNA into an animal-derived embryonic stem cell; and (3) culturing the embryonic stem cell obtained in step (2) into a clone, to obtain the cell strain or cell line.

In some embodiments, the sgRNA is selected from a group of sgRNAs that has a sequence having at least 85% or at least 90% or at least 91% or at least 92% or at least 93% or at least 94% or at least 95% or at least 96% or at least 97% or at least 98% or at least 99%, or 100% identity to the sequence as shown by SEQ ID NO: 1 or SEQ ID NO: 2.

In some embodiments, the animal is a mammal. In some embodiments, the animal is selected from a group consisting of pig, rat, mouse, hamster, rabbit, pig, bovine, deer, sheep, goat, chicken, cat, horse, dog, orangutan, and monkey. In some embodiments, the mammal is a rodent animal. In some embodiments, the rodent is a murine. In some embodiments, the animal is selected from an adult murine. In some embodiments, the animal is selected from a fetal murine. In some embodiments, the animal is an animal with a humanized gene.

In some embodiments, the gene is ACE2.

In some embodiments, the present disclosure provides an ACE2 gene humanized animal cell strain or cell line prepared by the method.

In some embodiments, the cell is a humanized mouse embryonic stem cell.

In some embodiments, the present disclosure provides a method for preparing a non-human animal. The method uses the above-described set of primers.

In some embodiments, the preparation method comprises a step of using the targeting vector.

In some embodiments, the method comprises a step of injecting the cell into an animal.

In some embodiments, the animal is a mammal. In some embodiments, the animal is selected from a group consisting of pig, rat, mouse, hamster, rabbit, pig, bovine, deer, sheep, goat, chicken, cat, horse, dog, orangutan, and monkey. In some embodiments, the mammal is a rodent animal. In some embodiments, the rodent is a murine. In some embodiments, the animal is selected from an adult murine. In some embodiments, the animal is selected from a fetal murine. In some embodiments, the animal is an animal with a humanized gene.

In some embodiments, the gene is ACE2.

In some embodiments, the present disclosure provides a non-human animal prepared by the above-described method.

In some embodiments, the animal is a mammal. In some embodiments, the animal is selected from a group consisting of pig, rat, mouse, hamster, rabbit, pig, bovine, deer, sheep, goat, chicken, cat, horse, dog, orangutan, and monkey. In some embodiments, the mammal is a rodent animal. In some embodiments, the rodent is a murine. In some embodiments, the animal is selected from an adult murine. In some embodiments, the animal is selected from a fetal murine. In some embodiments, the animal is an animal with a humanized gene.

In some embodiments, the gene is ACE2.

In some embodiments, the humanized mouse embryonic stem cell comprises human ACE2 gene. The humanized mouse embryonic stem cell can express human full-length ACE2 protein after specific differentiation or preparation into a mouse model, while the expression of endogenous ACE2 protein is decreased or lost.

In some embodiments, the present disclosure provides tissues, body fluids, cells, and debris or extracts thereof, of a humanized mouse or its offspring prepared by the above-described method.

In some embodiments, the present disclosure provides use of a humanized animal model or its offspring obtained by the above method in preparation of a human antibody, or as a model for pharmacological, immunological, microbiological and medical research, or for etiology research and/or for development of a new diagnostic and/or therapeutic strategy by preparing and using a laboratory animal disease model, or for screening, verification, evaluation or study of ACE2 gene function, an ACE2 antibody, a medicament targeting ACE2 and efficacy thereof.

### Brief Description of the Drawings

Fig. 1 schematically shows the plasmid pX330 map.
Fig. 2 shows the sequencing result of the construct pX330-sgRNA1.
Fig. 3 shows the sequencing result of the construct pX330-sgRNA2.
Fig. 4 shows the verification results of the cleavage efficiency of pX330-sgRNA1-3.
Fig. 5 shows the sequencing result of the construct pX330-sgRNA3.
Fig. 6 schematically shows the targeting strategy for humanized ACE2.
Fig. 7 shows the sequencing results of the humanized ACE2 targeting vector.
Fig. 8 shows the PCR identification results of the ligation of the three fragments in Comparative Example 1.
Fig. 9 shows the PCR identification results of the genotypes of the mouse embryonic stem cells having humanized ACE2.
Fig. 10 shows the PCR identification results of the genotypes of the mouse embryonic stem cells having humanized ACE2 after the deletion of PGK-Puro.
Fig. 11 shows the schematic diagram of the humanized ACE2 gene.
Fig. 12 shows the photograph of the humanized ACE2-genetically engineered mice obtained in Example 8.
Fig. 13 shows the qPCR detection results of the expression levels of hACE2 in the mice having humanized ACE2. The results show that mice tissues having humanized ACE2 specifically express hACE2, while the wild-type mice do not express hACE2.
Fig. 14 shows the qPCR detection results of the expression levels of mACE2 in the mice having humanized ACE2. The results show the hACE2 mice lack the expression of mACE2.
Fig. 15 shows the western blotting results of the expression of hACE2 protein in the intestinal tissues of the hACE2 mice and wild-type ACE2 mice. The results show that the hACE2 mice express hACE2 at the protein level.
Fig. 16 shows a flow chart of preparing the mice in Example 10, Test Example 1, Example 11, and Test Example 2.
Fig. 17 shows a flow chart of preparing the mice in Example 12, Test Example 3, Example 13, and Test Example 4.
Fig. 18 shows the results of the birth rates of the mice prepared in Examples 10-13.
Fig. 19 shows the results of the birth rates of the mice prepared in Test Examples 1-4.

### Detailed Description of the Embodiments

Technical solutions of the present disclosure are further described below by way of specific embodiments, which do not constitute limitations on the protection scope of the present disclosure. Non-essential modifications and adjustments made by other artisans based on the concepts of the present disclosure still fall within the protection scope of the present disclosure.

Unless otherwise defined, all technical and scientific terms used herein have the same definitions as those familiar to those skilled in the art. In addition, any methods and materials similar or equivalent to those described herein can be used in methods of the present disclosure. Preferred methods and materials are described in Detailed Description of the Embodiments.

"A" and "an" as used herein refer to the grammatical definition of the indefinite article, meaning "one", "one type of', or "a plurality of', "a variety of" (i.e., "at least one", "at least one type of'). For example, "an element" refers to one or more elements.

"CDS" is an abbreviation for coding sequence. The term "coding sequence" refers to any nucleotide sequence encoding the polypeptide product of a gene. In contrast, the term "non-coding sequence" refers to any nucleotide sequence that does not encode a polypeptide product of a gene.

The term "fragment" will be understood to refer to a nucleotide sequence that is shorter in length than a reference nucleic acid and shares a common nucleotide sequence with the reference nucleic acid. If appropriate, such a nucleic acid fragment according to the present disclosure can be included in a longer nucleotide sequence, and constitutes a part of the longer nucleotide sequence. Such fragments include, or consist of, oligonucleotides having a length in a range of at least 6, 8, 9, 10, 12, 15, 18, 20, 21, 22, 23, 24, 25, 30, 39, 40, 42, 45, 48, 50, 51, 54, 57, 60, 63, 66, 70, 75, 78, 80, 90, 100, 105, 120, 135, 150, 200, 300, 500, 720, 900, 1000 or 1500 consecutive nucleotides of a nucleic acid of the present disclosure.

In this specification, unless otherwise required by the context, the words "comprise" and "include" will be understood to include listed steps or elements or a collection of steps and elements, but does not exclude any other steps or elements or any collection of steps and elements. That is, the words are open-ended.

"Corresponding" means: (a) a polynucleotide has a nucleotide sequence that is substantially identical or complementary to all or part of a reference nucleotide sequence, or a polynucleotide encodes an amino acid sequence that is completely identical to an amino acid sequence in a peptide or protein; or (b) a peptide or a polypeptide has an amino acid sequence that is substantially identical to an amino acid sequence in a reference peptide or protein.

The term "downstream" refers to a nucleotide sequence at 3' terminal of a reference nucleotide sequence. In particular, a downstream nucleotide sequence generally relates to a sequence after the transcription start site. For example, the translation initiation codon of a gene is located downstream of the transcription start site.

The term "upstream" refers to a nucleotide sequence at 5' terminal of a reference nucleotide sequence. In particular, an upstream nucleotide generally relates to a sequence at 5' terminal of a coding sequence or a transcription start site. For example, most promoters are located upstream of a transcription start site.

"Promoter" refers to a DNA sequence capable of controlling a coding sequence or the expression of functional RNAs. Generally, a coding sequence is located at 3' terminal of a promoter sequence. A promoter may be derived in its entirety from a natural gene, or be composed of different elements derived from different promoters found in nature, or even include a synthetic DNA fragment. Those skilled in the art will appreciate that different promoters can direct gene expression in different tissues or cell types, or at different stages of development, or in response to different environmental or physiological conditions. Promoters that cause a gene to be expressed in most cell types most of the time are generally referred to as "constitutive promoters". Promoters that cause a gene to be expressed in a particular cell type are generally referred to as "cell-specific promoters" or "tissue-specific promoters". Promoters that cause a gene to be expressed at a particular stage of development or cell differentiation are generally referred to as "development-specific promoters" or "cell differentiation-specific promoters". Promoters that are induced and thus cause a gene to be expressed, after cells are exposed to or treated with agents, biomolecules, chemicals, ligands, light, or the like that induce the promoters, are often referred to as "inducible promoters" or "regulatory promoters". It should also be appreciated that DNA fragments of different lengths may have the same promoter activity because in most cases exact boundaries of regulatory sequences are not fully defined.

The term "5' UTR" or "5' uncoding sequence" or "5' untranslated region (UTR)" refers to a DNA sequence located upstream (5') of a coding sequence.

The terms "restriction endonuclease" and "restriction enzyme" refer to enzymes that join and cleave a specific nucleotide sequence within a double-stranded DNA.

The term "vector" refers to a nucleic acid molecule capable of transferring a nucleic acid molecule linked thereto. One type of vector is "plasmid" which refers to a circular double-stranded DNA loop into which other DNA segments can be ligated. Another type of vector is viral vectors, by way of which other DNA segments can be ligated into viral genomes. Some vectors are capable of self-replication in host cells into which they are introduced (e.g., bacterial vectors with bacterial origins of replication, and episomal mammalian vectors). Other vectors (e.g., non-episomal mammalian vectors) are capable of integrating into the genome of host cells after being introduced into the host cells and thus replicate with the host genome. In addition, some vectors are capable of directing the expression of genes to which they are operably linked.

Some vectors, referred to in the present disclosure as "recombinant expression vectors" (or simply "expression vectors"), are vectors, plasmids or media designed to enable expression of an inserted nucleic acid sequence upon it being transferred to a host. In general, expression vectors used in recombinant DNA technology are often in the form of plasmids. In this specification, "plasmid" and "vector" are used interchangeably because plasmids are the most commonly used form of vectors. However, the present disclosure is intended to include other forms of such expression vectors, such as viral vectors (e.g., replication-defective retroviruses, adenoviruses, and adeno-associated viruses), which serve equivalent functions.

The term "plasmid" refers to an extrachromosomal element that often carries genes that are not part of the central metabolism of a cell and is often in the form of a circular double-stranded DNA molecule. Such elements can be autonomously replicating sequences, genomic integration sequences, phage or nucleotide sequences, linear, circular or supercoiled, single-stranded or double-stranded DNAs or RNAs from any source, among which many nucleotide sequences have been ligated or recombined into a unique construct that is capable of introducing a promoter fragment and a DNA sequence as well as an appropriate 3'-terminal untranslated sequence for a selected gene product into cells.

A "targeting vector" or "target vector" is a DNA construct that contains sequences "homologous" to endogenous chromosomal nucleic acid sequences flanking a desired genetic modification. The flanking homology sequences (referred to as "homology arms") direct the targeting vector to a specific chromosomal location within the genome by virtue of the homology that exists between the homology arms and the corresponding endogenous sequence and introduce the desired genetic modification by a process referred to as "homologous recombination". "Targeting vector" and "target vector" can sometimes be used interchangeably. A targeting vector is used to introduce an inserted nucleic acid into a targeted locus in a nucleic acid of a rat, a eukaryotic animal, a non-rat eukaryotic animal, a mammal, a non-human mammal, a human, a rodent, a non-rat rodent, a mouse, or a hamster. The targeting vector comprises the inserted nucleic acid and further comprises a 5' homology arm and a 3' homology arm flanking the inserted nucleic acid. The homology arms flanking the inserted nucleic acid correspond to regions within the targeted locus in the nucleic acid of the rat, the eukaryotic animal, the non-rat eukaryotic animal, the mammal, the non-human mammal, the human, the rodent, the non-rat rodent, the mouse, or the hamster. For ease of reference, a corresponding homologous genomic region within a targeted genomic locus is referred to herein as a "target site". For example, a targeting vector may comprise a first inserted nucleic acid flanked by first and second homology arms complementary to first and second target sites. The targeting vector thus facilitates the integration of the inserted nucleic acid into the targeted locus in the nucleic acid of the rat, the eukaryotic animal, the non-rat eukaryotic animal, the mammal, the non-human mammal, the human, the rodent, the non-rat rodent, the mouse, or the hamster by virtue of homologous recombination events that occur between the homology arms and the complementary target sites within the genome of cells.

In one embodiment, the targeted locus in the nucleic acid of the rat, the eukaryotic animal, the non-rat eukaryotic animal, the mammal, the non-human mammal, the human, the rodent, the non-rat rodent, the mouse, or the hamster comprises and a first nucleic acid sequence complementary to the 5' homology arm and a second nucleic acid sequence complementary to the 3' homology arm.

A vector can be introduced into desired host cells by methods known in the art, such as transfection, electroporation, microinjection, transduction, cell fusion, DEAE dextran, calcium phosphate precipitation, lipofection (lysosomal fusion), use of a gene gun or a DNA vector transporter (see e.g., Wu et al., 1992, J. Biol. Chem. 267:963-967; Wu and Wu, 1988, J. Biol. Chem. 263:14621-14624; and Hartmut et al., Canadian Patent Application 2,012,311, filed on March 15, 1990).

The term "transfection" refers to the uptake of an exogenous or heterologous RNA or DNA by a cell. When an exogenous or heterologous RNA or DNA has been introduced into a cell, the cell is "transfected" with such RNA or DNA. When the transfected RNA or DNA affects a phenotypic change, the cell is "transformed" with the exogenous or heterologous RNA or DNA. The transforming RNA or DNA can be integrated (covalently linked) into the chromosomal DNA that makes up the genome of the cell.

The term "homology" or "homologous" means that two sequences, e.g., nucleotide or amino acid sequences, when optimally aligned and compared, are identical in at least about 75% of the nucleotides or amino acids, at least about 80% of the nucleotides or amino acids, at least about 90-95% of the nucleotides or amino acids, e.g., more than 97% of the nucleotides or amino acids. Those skilled in the art will appreciate that for optimal gene targeting, a targeting construct should contain arms (i.e., "homology arms") that are homologous to endogenous DNA sequences. Homologous recombination can therefore occur between the targeting construct and the targeted endogenous sequences.

As used herein, a homology arm and a target sequence (i.e., homologous genomic regions) are complementary to each other when the two regions share a sufficient level of sequence identity to each other to act as substrates for a homologous recombination reaction. "Homology" means that a DNA sequence is either identical to or shares sequence identity with a corresponding or "complementary" sequence. The sequence identity between a given target site and a corresponding homology arm found on the targeting vector can be any degree of sequence identity that allows homologous recombination to occur. For example, the amount of sequence identity shared by the homology arm of the targeting vector (or a fragment thereof) and the target site (or a fragment thereof) may be at least 51%, 53%, 57%, 60%, 65%, 70% , 75%, 80%, 83%, 85%, 87%, 89%, 91%, 93%, 95%, 97%, 98%, 99% or 100% sequence identity, such that the sequences undergo homologous recombination. Moreover, a complementary region of homology between the homology arm and the complementary target site can be of any length that is sufficient to promote homologous recombination at the cleaved recognition site. The homology arm thus has sufficient homology with the corresponding target site within the genome of the cell to undergo homologous recombination. For ease of reference, the homology arms are referred to herein as 5' homology arm and 3' homology arm. This terminology refers to the relative position of homology arms to an inserted nucleic acid within a targeting vector.

In some embodiments, a homology arm of a targeting vector can be of any length that is sufficient to promote a homologous recombination event with a corresponding target site, for example, being at least 5-10 kb, 5-15 kb, 10-20 kb, 20-30 kb , 30-40 kb, 40-50 kb, 50-60 kb, 60-70 kb, 70-80 kb, 80-90 kb, 90-100 kb, 100-110 kb, 110-120 kb, 120-130 kb, 130-140 kb, 140-150 kb, 150-160 kb, 160-170 kb, 170-180 kb, 180-190 kb, 190-200 kb in length or greater. As described in further detail below, a targeting vector may employ a targeting arm of a greater length. In a particular embodiment, a sum total of 5' and 3' homology arm is at least 10 kb or at least about 16 kb to about 100 kb or about 30 kb to about 100 kb. In other embodiments, a sum total of 5' and 3' homology arms of ACE2 is about 10 kb to about 150 kb, about 10 kb to about 100 kb, about 10 kb to about 75kb, about 20 kb to about 150 kb , about 20 kb to about 100 kb, about 20 kb to about 75kb, about 30 kb to about 150 kb, about 30 kb to about 100 kb, about 30 kb to about 75kb, about 40 kb to about 150 kb, about 40 kb to about 100 kb, about 40 kb to about 75kb, about 50 kb to about 150 kb, about 50 kb to about 100 kb, or about 50 kb to about 75kb, about 10 kb to about 30 kb, about 20 kb to about 40 kb, about 40 kb to about 60 kb, about 60 kb to about 80 kb, about 80 kb to about 100 kb, about 100 kb to about 120 kb, or about 120 kb to about 150 kb.

Some embodiments herein relate to a humanized gene-edited mammal, the genome of which comprises a polyribonucleic acid encoding a human full-length ACE2 protein. For example, the polyribonucleic acid is operably linked to a promoter polyribonucleic acid. In some embodiments, the humanized gene-edited mammal does not express all or part of the polyribonucleic acid encoding the endogenous ACE2 protein of the humanized gene-edited mammal, and the polyribonucleic acid encoding the human ACE2 protein comprises modifications of the human ACE2 protein gene.

In some embodiments, the cells are pluripotent cells, non-pluripotent cells, mammalian cells, human cells, non-human mammalian cells, rodent cells, mouse cells, hamster cells, non-human pluripotent cells, human pluripotent cells, rodent pluripotent cells, or fibroblasts cells, or lung cells.

In some of the above methods, the cells are primary cells or immortalized cells. In some of the above methods, the rodent pluripotent cells are mouse or rat embryonic stem (ES) cells.

In some of the above methods, the animal cells or the human cells are primary cells or immortalized cells. In some of the above methods, the animal cells or the human cells are pluripotent cells. In some of the above methods, the animal pluripotent cells are mouse embryonic stem (ES) cells. In some of the above methods, the human pluripotent cells are human embryonic stem (ES) cells, human adult stem cells, developmentally restricted human progenitor cells, or human induced pluripotent stem (iPS) cells.

In some embodiments, some examples provide humanized gene-edited cells, and provide, in particular, isolated human and non-human totipotent or pluripotent stem cells, in particular mouse embryonic stem cells, which are capable of maintaining pluripotency after one or more in vitro sequential genetic modifications and are capable of passing the targeted genetic modifications onto offspring through germline.

The term "embryonic stem cells" or "ES cells" as used herein include embryo-derived totipotent or pluripotent cells capable of promoting the development of any tissue of an embryo after being introduced into the embryo. The term "pluripotent cells" as used herein include undifferentiated cells that have the ability to develop into more than one type of differentiated cells. The term "non-pluripotent cells" include cells that are not pluripotent cells.

In some of the above methods, targeted gene editing also includes deletion of an endogenous nucleic acid sequence from a genomic locus of interest or insertion of a nucleic acid into the genomic locus of interest.

In some embodiments, genetic modifications or gene editing include two or more independent modifications of cells (e.g., eukaryotic cells, non-rat eukaryotic cells, mammalian cells, cell-like cells, non-human mammalian cells, pluripotent cells, non-pluripotent cells, non-human pluripotent cells, human pluripotent cells, human ES cells, human adult stem cells, developmentally restricted human progenitor cells, human iPS cells, human cells, rodent cells, non-rat rodent cells, rat cells, mouse cells, hamster cells, fibroblasts, or Chinese hamster ovary (CHO) cells). A first modification can be achieved by electroporation or any other method known in the art. Subsequently, a second modification of the genome of a same cell is performed using a suitable second nucleic acid construct. A third modification can be achieved by a second electroporation or any other method known in the art. In various embodiments, following a first genetic modification and a second genetic modification of a same cell, it is possible to perform a third genetic modification, a fourth genetic modification, a fifth genetic modification, a sixth genetic modification, etc. (one genetic modification followed by another genetic modification) using, for example, continuous electroporation, or any other methods known in the art (continuously).

In some embodiments of the present disclosure, gene editing is performed by using a targeting vector through homologous recombination process, thereby inserting an exogenous nucleic acid into an endogenous genome.

In some embodiments, inserting a nucleic acid includes insertion of a homologous or orthologous human nucleic acid sequence, or replacement of a nucleic acid sequence of a eukaryotic, non-rat eukaryotic, mammalian, human, or non-human mammalian animal with the homologous or orthologous human nucleic acid sequence.

In some embodiments, a given insert polynucleotide can be from any organism, including, for example, rodents, non-rat rodents, rats, mice, hamsters, mammals, non-human mammals, eukaryotes , non-rat eukaryotes, humans, agricultural animals, or domestic animals.

In certain embodiments, the inserted nucleic acid may comprise a nucleic acid from a rat, which may comprise a fragment of a genomic DNA, a cDNA, a regulatory region, or any portion, or a combination thereof. In other embodiments, the inserted nucleic acid may comprise a nucleic acid from an eukaryote, a non-rat eukaryote, a mammal, a human, a non-human mammal, a rodent, a non-rat rodent, a human, a rat, a mouse, a hamster, a rabbit, a pig, a bovine, a deer, a sheep, a goat, a chicken, a cat, a dog, a ferret, a primate (e.g., a marmoset, a rhesus monkey), a domesticated or agricultural mammal, or any other organism of interest. As described in more detail herein, the insert nucleic acid employed in the various methods and compositions can cause "humanization" of the targeted locus of interest.

In some embodiments, a genetic modification is addition of a nucleic acid sequence. In one or more embodiments, an insert nucleic acid comprises a genetic modification in a coding sequence. In some embodiments, genetic modifications include a deletion mutation of a coding sequence. In some embodiments, genetic modifications include the fusion of two endogenous coding sequences. In some embodiments, inserting a nucleic acid includes insertion of a homologous or orthologous human nucleic acid sequence, or replacement of a nucleic acid sequence of a eukaryotic, non-rat eukaryotic, mammalian, human, or non-human mammalian animal with the homologous or orthologous human nucleic acid sequence. In some embodiments, inserting a nucleic acid includes the insertion of a homologous or orthologous human nucleic acid sequence into an endogenous mouse gene coding region containing a corresponding mouse DNA sequence or replacement of the mouse DNA sequence with the homologous or orthologous human nucleic acid sequence. In some embodiments, inserting a nucleic acid includes the insertion of a homologous or orthologous human nucleic acid sequence into an endogenous mouse gene coding region containing a corresponding mouse DNA sequence or replacement of the mouse DNA sequence with the homologous or orthologous human nucleic acid sequence. In some embodiments, a targeting vector is used to insert, into the mouse ACE2 locus, a hACE2 sequence immediately after an EXON1 CDS initiation ATG site of mACE2.

In some embodiments, a nucleic acid sequence of a targeting vector may comprise a polynucleotide that, when integrated into a genome, will generate a genetic modification of a region at an ACE2 locus of a mammal, a human, or a non-human mammal. The genetic modification at the ACE2 gene locus result in decreased ACE2 activity, increased ACE2 activity, or adjustment of ACE2 activity. In some embodiments, the ACE2 gene is completely replaced.

In some embodiments, an inserted nucleic acid may comprise a regulatory element, including, for example, a promoter, an enhancer, or transcription factor.

In some embodiments, a given inserted polynucleotide and/or a corresponding replacement region of a locus of a mammal, a human cell, or a non-human mammal may be a coding region, an intron, an exon, an untranslated region, a regulatory region, a promoter or an enhancer, or any combination thereof.

Provided herein are methods that allow for integration of one or more polynucleotides of interest into a target locus, as outlined above, thereby producing gene-edited cells by introducing a sequence. By "introducing", a sequence (polypeptide or polynucleotide) is delivered into a cell by means of the entry of the sequence into the cell.

Any cells from any organism can be used in the methods provided herein. In certain embodiments, the cells are from a eukaryote, a non-rat eukaryote, a mammal, a non-human mammal, a human, a rodent, a non-rat rodent, a rat, a mouse, or a hamster. In certain embodiments, the cells are eukaryotic cells, non-rat eukaryotic cells, pluripotent cells, non-pluripotent cells, non-human pluripotent cells, non-human mammalian cells, human pluripotent cells, human ES cells, human adult stem cells, developmentally restricted human progenitor cells, human induced pluripotent (iPS) cells, mammalian cells, human cells, fibroblasts, rodent cells, non-rat rodent cells, rat cells, mouse cells, mouse ES cells, hamster cells, or CHO cells.

In some embodiments, cells employed in the methods have a DNA construct stably incorporated into their loci. "Stably incorporated" or "stably introduced" refers to introduction of a polynucleotide into a cell, such that the nucleotide sequence is integrated into the genome of the cell and can be inherited to its offspring.

In some embodiments, one or more polynucleotides are introduced into cells by electroporation, intracytoplasmic injection, viral infection, adenovirus, lentivirus, retrovirus, transfection, lipid-mediated transfection, or Nucleofection^{™}-mediated transfection.

In some embodiments, an expression construct is introduced together with an introduced nucleic acid.

In some embodiments, the introduction of one or more polynucleotides into the cells can be performed multiple times over a period of time. In some embodiments, the introduction of one or more polynucleotides into the cells can be performed at least twice over a period of time, at least three times over a period of time, at least four times over a period of time, at least five times over a period of time, at least six times over a period of time, at least seven times over a period of time, at least eight times over a period of time, at least nine times over a period of time, at least ten times over a period of time, at least eleven times over a period of time, at least twelve times over a period of time, at least thirteen times over a period of time, at least fourteen times over a period of time, at least fifteen times over a period of time, at least sixteen times over a period of time, at least seventeen times over a period of time, at least eighteen times over a period of time, at least nineteen times over a period of time, or at least twenty times over a period of time.

In some embodiments, a targeting vector (containing an introduced nucleic acid) is introduced into cells simultaneously with an expression vector (containing sgRNA).

In some embodiments, provided further is a method for preparing a humanized non-human animal. The method includes: (a) modifying a genome of a pluripotent cell with a targeting vector comprising an inserted nucleic acid to form a donor cell, the inserted nucleic acid comprising a human nucleic acid sequence; (b) introducing the donor cell into a host embryo; and (c) incubating the host embryo in a surrogate mother, so that the surrogate mother produces offspring containing the human nucleic acid sequence. In some embodiments, the donor cell is introduced into the host embryo at blastocyst stage or at premorula stage (i.e., at 4-cell stage or 8-cell stage). In still further embodiments, the genetic modification can be transmitted via germline.

In certain embodiments, provided is a method for preparing a humanized mouse. The method includes: (a) introducing a targeting vector comprising a fragment of ACE2 gene and an expression vector linked with an sgRNA into a mouse embryonic cell to form a gene-edited donor cell; (b) introducing the donor cell into a mouse embryo; and (c) incubating the mouse embryo in a surrogate mother, so that the surrogate mother produces offspring containing the human ACE2 sequence.

In this text, the terms "tetraploid complementation technique" and "tetraploid embryo complementation technique" or "tetraploid complementation assay" or "tetraploid embryo complementation assay" can be equivalent, and have the following meaning. When a certain number of embryonic stem (ES) cells and a tetraploid embryo are chimerized, during the development of the chimera, the ES cells and the tetraploid embryo are not randomly distributed. The tetraploid embryo only participates in the formation of extraembryonic tissues such as yolk sac endoderm and placental trophoblast cells (such as chorionic ectoderm, trophoblast cells, etc.), while the ES cells are extensively involved in the generation of embryoid body, allantois, amniotic membrane, yolk sac mesoderm and chorionic mesoderm, but not involved in the generation of yolk sac endoderm and placental trophoblast cell lineage. In other words, developmental potencies of the two are complementary, which phenomenon is called tetraploid complementation.

In some cases, the term "embryo" refers to an animal subject at any time before birth or tissues and cells derived from an animal subject at any time before birth.

In some instances, the term "embryo" is used to describe a fertilized oocyte that has been implanted into a uterus before becoming a fetus eight weeks after fertilization. According to this definition, a fertilized oocyte is often referred to as a pre-embryo prior to transplantation. However, throughout this disclosure, a broader definition for the term "embryo", covering the pre-embryonic stage, will be used. The term thus covers all developmental stages from oocyte fertilization to morula, blastosphere stage hatching, and implantation.

An embryo is near-spherical and consists of one or more cells (blastomere) surrounded by a gelatin-like outer shell, with an acellular matrix known as zona pellucida. The zona pellucida serves several functions until the embryo hatches and is a good marker for embryo evaluation. The zona pellucida is spherical and translucent, and should be clearly distinguished from cellular debris.

A mammalian preimplantation embryo is about 90-120 microns in diameter, surrounded by clearly visible zona pellucida composed of glycoproteins. On one side of the zygote, there are 1-2 polar bodies formed during meiosis.

Between 23-43.5 hours after fertilization, the ovum undergoes a first mitotic division and forms an embryo with 2 blastomeres. Cells of the 2-cell embryo are nearly oval in shape, and are substantially the same in size. After application of an electrical field, the two blastomeres can fuse into a tetraploid cell and then develop into a tetraploid embryo. The fertilized ovum divides into two, forming two blastomeres, which is the 2-cell stage. Further, the two blastomeres each divide into two, forming altogether four cells, which the 4-cell stage. The four cells then each divide into two, forming altogether eight cells, which is the 8-cell stage. When 32 cells are formed after 5 divisions, a "morula" is formed. At 4-cell stage, the 4 blastomeres are arranged in a staggered manner. At 8-cell stage, the blastomeres are arranged in two layers, and start to undergo compaction with the formation of tight junctions between the layers. Starting from 16-cell stage, the embryo is called a morula, at which point differentiation of inner cell mass and trophoblast occurs. At 72 hours after fertilization, the embryo develops to 32-cell stage, and a gap appears between the blastomeres, which then increases to form a complete blastocoel. On one side of the blastocoel, there is an accumulation of cells, which is the inner cell mass. A layer of flat cells surrounding the blastocoel is called trophoblast. The blastocyst at this time is also called blastosphere. The blastosphere later expands and escapes the zona pellucida, i.e., the blastosphere hatches.

The term "embryo" is used to denote the following stages: zygote metrocyte, zygote, 2-cell stage, 4-cell stage, 8-cell stage, 16-cell stage, morula, blastosphere, expanded blastosphere, and hatched blastosphere, and all stages in between (e.g., 3-cell or 5-cell stage).

The term "chimeric blastocyst" or "chimeric embryo" as used herein refers to a blastocyst or embryo comprising embryonic stem cells and in a chimeric state. The chimeric blastocyst or embryo can be produced using, for example, the so-called "aggregation method", in addition to the injection method. In the "aggregation method", embryo+embryo, or embryo+cells are enabled to closely adhere to each other in a culture dish to produce a chimeric blastocyst. Furthermore, a recipient blastocyst or embryo and cells to be transplanted can be in an allogeneic relationship or a xenogeneic relationship.

As used herein, the term "give" or "transplant" refers to the placement of cells into a subject by a method or approach of localizing the cells at least partially to a desired site to thus produce a desired effect.

### Example 1. sgRNAl for ACE2 gene and construction of pX330-sgRNA plasmid

An sgRNA1 sequence capable of recognizing the target site was synthesized.

sgRNA1 sequence (SEQ ID NO: 1): 5'-tactgctcagtccctcaccgagg-3'.

The upstream and downstream annealing primers for the sgRNA were synthesized by introducing a BbsI cut site for the sgRNA, and then subjected to the subsequent annealing experiments. The upstream and downstream single-stranded primers having the following sequences were synthesized for the sgRNA1:
upstream: 5'-caccgcttggcattttcctcggtga-3' (SEQ ID NO:4), and
downstream: 5'-aaactcaccgaggaaaatgccaagc-3' (SEQ ID NO:5).

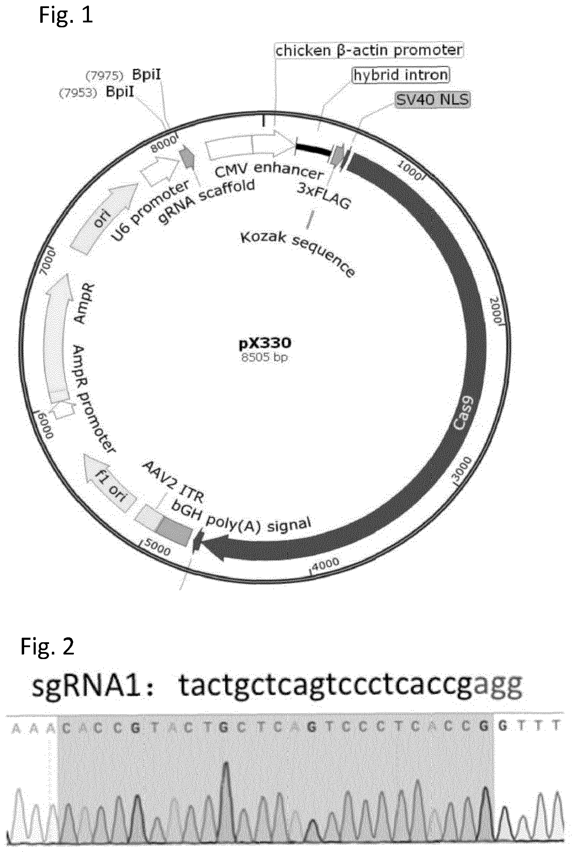

The source of pX330 plasmid: see Fig. 1 for pX330 vector map. The plasmid backbone was obtained from MiaoLing Plasmid Platform with Cat. No. P0123.

The above sgRNA annealing primers, after annealing, were ligated to the pX330 plasmid (the plasmid had been linearized with BbsI) to obtain the expression vector pX330-sgRNA1.

The ligation reaction system is specifically shown in Table 1.

**Table 1. Ligation reaction system**

| | |
|---|---|
| sgRNA annealing products | 1 µL (0.5 µM) |
| pX330-sgRNA vector | 1 µL (20 ng) |
| T4 DNA Ligase | 1 µL (5 U) |
| 10×T4 DNA Ligase buffer | 1 µL |
| H₂O | Up to 10 µL |

The reaction conditions were as follows: incubating at 16°C for more than 30 min for the ligation. The reaction was transformed into 30 µL TOP 10 competent cells. 200 µL of the cells were then taken and applied to an ampicillin (Amp)-resistant plate, and cultured at 37°C for at least 12 hours. Then, 2 clones were selected and inoculated in Amp-resistant LB medium (5 mL), and subjected to shaking cultivation at 37°C, 250 rpm, for at least 12 hours.

The randomly selected clones were sent to a sequencing company for sequencing verification. The sequencing result is shown in Fig. 2. The expression vector pX330-sgRNA1 having the correct ligation was selected for subsequent experiments.

### Example 2. sgRNA2 for ACE2 gene and construction of pX330-sgRNA2 plasmid

An sgRNA2 sequence capable of recognizing the target site was synthesized.

sgRNA2 sequence (SEQ ID NO: 2): 5'-cttggcattttcctcggtgaggg-3'

The upstream and downstream annealing primers for the sgRNA were synthesized by introducing a BbsI cut site for the sgRNA, and then subjected to the subsequent annealing experiments. The upstream and downstream single-stranded primers having the following sequences were synthesized for the sgRNA2:
upstream: 5'-caccgtactgctcagtccctcaccg-3' (SEQ ID NO:6), and
downstream: 5'-aaaccggtgagggactgagcagtac-3' (SEQ ID NO:7).

The source of pX330 plasmid: see Fig. 1 for pX330 vector map. The plasmid backbone was obtained from MiaoLing Plasmid Platform with Cat. No. P0123.

The above sgRNA annealing primers, after annealing, were ligated to the pX330 plasmid (the plasmid had been linearized with BbsI) to obtain the expression vector pX330-sgRNA2.

The ligation reaction system is specifically shown in Table 2.

**Table 2. Ligation reaction system**

| | |
|---|---|
| sgRNA annealing products | 1 µL (0.5 µM) |
| pX33 0-sgRNA vector | 1 µL (20 ng) |
| T4 DNA Ligase | 1 µL (5 U) |
| 10×T4 DNA Ligase buffer | 1 µL |
| H₂O | Up to 10 µL |

The reaction conditions were as follows: incubating at 16°C for more than 30 min for the ligation. The reaction was transformed into 30 µL TOP10 competent cells. 200 µL of the cells were then taken and applied to an Amp-resistant plate, and cultured at 37°C for at least 12 hours. Then, 2 clones were selected and inoculated in Amp-resistant LB culture medium (5 mL), and then subjected to shaking cultivation at 37°C, 250 rpm, for at least 12 hours.

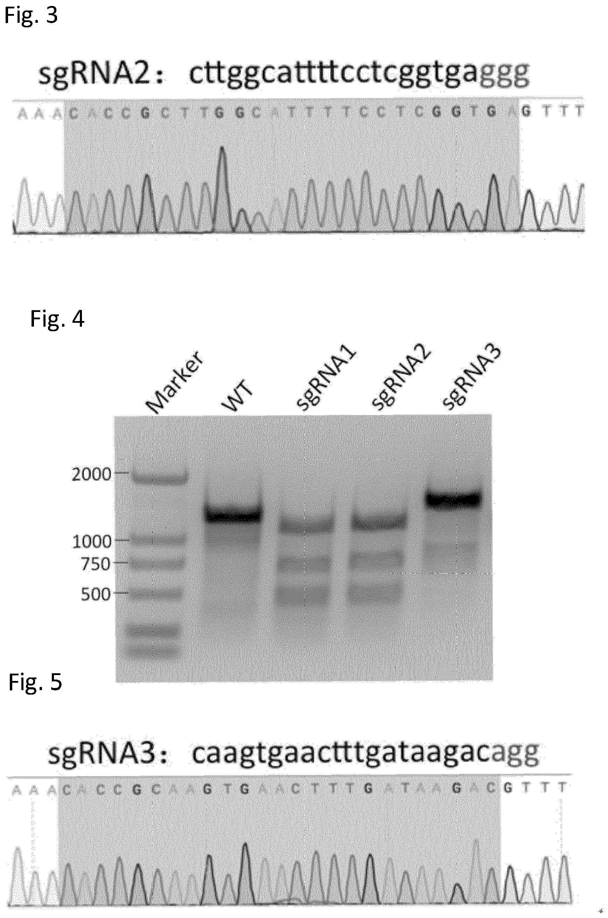

The randomly selected clones were sent to a sequencing company for sequencing verification. The sequencing result is shown in Fig. 3. The expression vector pX330-sgRNA2 having the correct ligation was selected for subsequent experiments.

### Example 3. Evaluation of pX330-sgRNA cleavage efficiency

2 µg of each of the pX330-sgRNA plasmids prepared in Examples 1 and 2 was transfected into mouse embryonic stem cells by Lipofectamine 3000 (Lipofectamine 3000, Invitrogen, Cat. No. L3000001) according to Lipofectamine 3000 reagent protocol. Two days after the transfection, the mouse embryonic stem cells were harvested for genomic DNA extraction by means of a cell genomic DNA extraction kit (Tiangen, DP304-02). Then, the upstream primer (5arm-sgF: ggttttgatttggccataaaatgttagc (SEQ ID NO: 10)) and downstream primer (3arm-sgR: attcccaggtccagtttcacctaag (SEQ ID NO: 11)) were designed flanking the genomic cleavage site. Then, a PCR reaction was performed by using the upstream and downstream primers for the extracted genome (Novozan Phanta Max Super-Fidelity DNA Polymerase). Then, the cleavage efficiency of pX330-sgRNAs were assessed by using T7 endonuclease I (T7EI) (Biolabs, M0302L), which could recognize and cleave non-perfectly matched DNA.

The T7 E7 experiment was performed following the specific steps below.

Purify genomic DNA from the above cells transfected with pX330-sgRNA1 and pX330-sgRNA2, respectively, and amplify with primers 5arm-sgF+3arm-sgR to obtain the PCR product (Tiangen, DP214-02); anneal 1 µg of the PCR product in the following reaction.

**Table 3**

| | |
|---|---|
| PCR product | 1 µg |
| Buffer 2 | 2 µL |
| H₂O | Up to 20 µL |
| Reaction conditions | Boil in a water bath for 10 min and then cool naturally |

Then, 1 µL of T7 endonuclease I was added to the annealed product for incubating at 37°C for 30 min, and then the reaction was directly verified by means of gel electrophoresis. The results are shown in Fig. 4. sgRNA1 and sgRNA2 prepared in Examples 1 and 2, respectively, were cleaved into fractions with higher brightness, (sgRNA1 was cleaved into two bands of about 376 bp and 581 bp, and sgRNA2 was cleaved into two bands of about 371 bp and 586 bp). It showed that the cleavage efficiency of sgRNA1 and sgRNA2 was relatively high.

Since the cleavage efficiency is mainly revealed through images, Fig. 4 shows the cleavage efficiency of another sgRNA (referred to as sgRNA3), in order to show the technical effects of the solutions of Examples 1 and 2.
sgRNRA3 sequence (SEQ ID NO:3): 5'-caagtgaactttgataagacagg-3'

The upstream and downstream single-stranded primers having the following sequences were synthesizing for the sgRNA3:
upstream: 5'-caccgcaagtgaactttgataagac-3' (SEQ ID NO:8); and
downstream: 5'-aaacgtcttatcaaagttcacttgc-3' (SEQ ID NO:9).

The remaining steps were the same as those in Example 1. Clones were randomly selected and sent to a sequencing company for sequencing. The sequencing result is shown in Fig. 5. The expression vector pX330-sgRNA3 having the correct ligation was selected for subsequent experiments.

Since the other sgRNAs and the construction of pX330-sgRNA plasmids are not the focus of the present disclosure, they will not be repeated and listed in further detail.

### Example 4. Design of the targeting vector

According to human ACE2 (Gene ID: 59272) transcript with NCBI accession No. NM_001371415.1→NP_001358344.1, it was determined that the coding sequence (CDS) of ACE2 (hACE2-CDS) had a nucleotide sequence as shown by SEQ ID NO: 12, and ACE2 protein (hACE2-protein) had an amino acid sequence as shown by SEQ ID NO: 13. The hACE2-CDS was inserted downstream of a promoter and 5' UTR region of mouse ACE2, to initiate the expression of human ACE2 through the mouse ACE2 promoter. At the same time, a termination signal, SV40 polyA (SEQ ID NO: 14), was added after the inserted human ACE2 CDS sequence, to terminate the mRNA transcription of human ACE2 more efficiently.

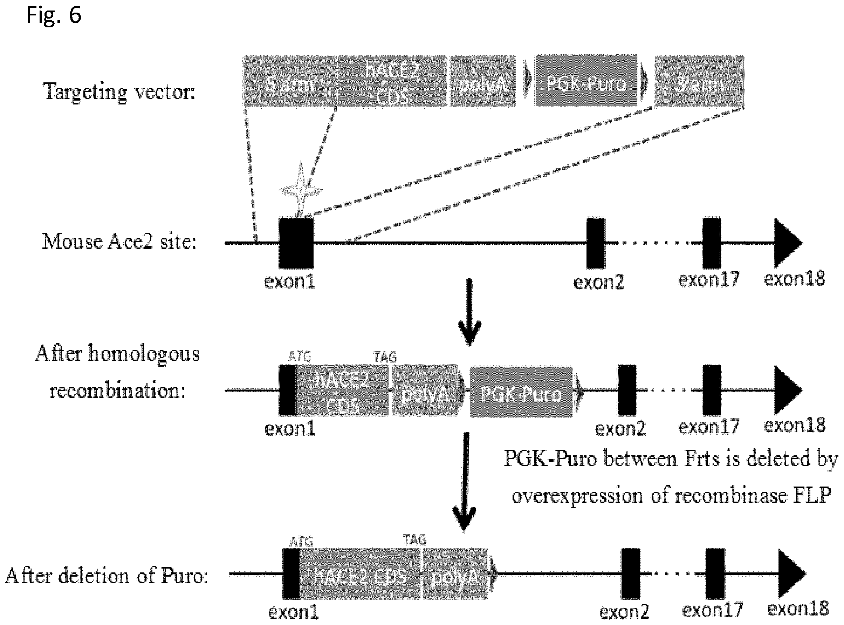

The inventors further designed a targeting scheme as shown in Fig. 6, as well as a vector comprising a 5' homology arm (5arm), a human ACE2 gene fragment, and a 3' homology arm (3arm). The 5' homology arm comprised nucleotides 125683-126652 of NCBI accession number AC091606.8, and had a nucleotide sequence as shown by SEQ ID NO: 15. The 3' homology arm comprises nucleotides 126796-127766 of NCBI accession number AC091606.8, and had a nucleotide sequence as shown by SEQ ID NO: 16. A PGK-puromycin exogenous expression cassette (PGK-puro, SEQ ID NO: 17) was inserted into the vector as a selection marker. Further, the PGK-puromycin expression cassette was flanked with a pair of FRT sites (SEQ ID NO: 18) in the same orientation, such that the gene could be removed using a FLP recombinase to solve safety issues caused by transgenosis. The constructed vector was verified through sequencing. The vector was linearized by AgeI before performing the targeting experiments. The schematic diagram of the targeting vector and targeting strategy are shown in Fig. 6.

The vector construction process included the following steps:
Design an upstream primer (5arm-pcrF: tcgcacacattccacatccaccggtccctatggagtggagaagagtctta (SEQ ID NO: 19)) and a matching downstream primer (5arm-pcrR: gaaggagccaggaagagcttgacatctttccccgtgcgccaagatcc (SEQ ID NO: 20)) for amplifying an overlapping fragment of the 5' homology arm.

Design an upstream primer (hACE2-F: ggatcttggcgcacggggaaagatgtcaagctcttcctggctccttc (SEQ ID NO: 21)) and a matching downstream primer (hACE2-R: cattataagctgcaataaacaagttctaaaaggaggtctgaacatcatc (SEQ ID NO: 22)) for amplifying an overlapping fragment of the human ACE2 CDS.

Design an upstream primer (SV40-F: gatgatgttcagacctccttttagaacttgtttattgcagcttataatg (SEQ ID NO: 23)) and a matching downstream primer (SV40-R: AGAGAATAGGAACTTCGCACGCGTtaagatacattgatgagtttggac (SEQ ID NO: 24)) for amplifying an overlapping fragment of SV40 polyA.

Design an upstream primer (3arm-pcrF: tacgaagttatGtcgacgcGGCGCGCCgaattataatactaacattactg (SEQ ID NO: 25)) and a matching downstream primer (3arm-pcrR: tatgaccatgattacgccaagcttaagaccaaactattagagcagttaaaagc (SEQ ID NO: 26)) for amplifying the 3' homology arm fragment. The template for the amplification of the 5' and 3' homology arms was C57BL6/J mouse genomic DNA. The template for the amplification of the human ACE2 was the cDNA derived from human lung cells. The PCR reaction (Novozymes Phanta Max Super-Fidelity DNA Polymerase) and conditions thereof are shown in Table 4.

**Table 4. PCR system (50 µL)**

| | |
|---|---|
| 2×Phanta Max Buffer | 25 µL |
| dNTP Mix | 1 µL |
| Upstream primers (10µM) | 2 µL |
| Downstream primers (10µM) | 2 µL |
| Phanta Max Super-Fidelity DNA Polymerase | 1 µL |
| Templates | 5' homology arm, human ACE2 CDS, SV40 polyA fragment each 50 ng |
| H₂O | Up to 50 µL |
| PCR amplification conditions | Start with 65°C, and decrease the temperature by 0.3°C each cycle |

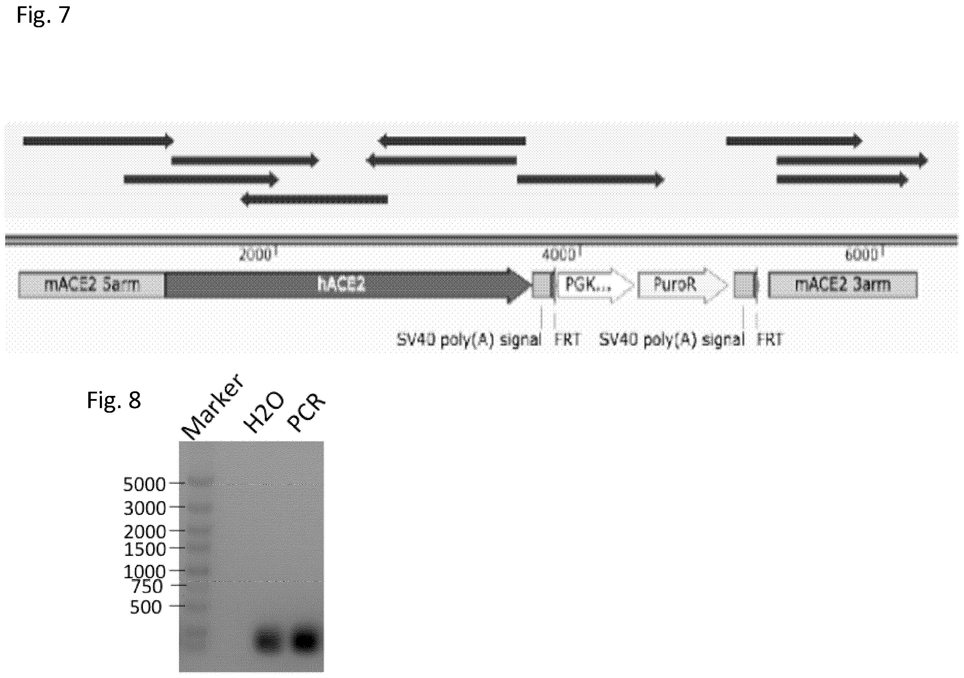

In this way, a continuous fragment 5arm-hACE-SV40 was obtained from the PCR amplification products, i.e., the 5' homology arm, the human ACE2 CDS, and the SV40 polyA, by means of the overlap PCR in conjunction with the touchdown PCR. The fragment 5arm-hACE-SV40 and the 3' homology arm obtained by PCR were recovered and then directly used for constructing a homologous recombination targeting vector. The construction process included the following steps.
1. Subject the 5arm-hACE-SV40 fragment to AgeI+MluI double digestion, subject the 3' homology arm fragment to AscI+HindIII double digestion, and then ligate the digested fragments respectively, so as to obtain the targeting vector.
2. Identify positive targeting vector by enzyme digestion, and then perform sequencing verification for the identified targeting vector by the sequencing company. The sequencing result showed the targeting vector had the correct sequence (as shown in Fig. 7). In this way, the humanized ACE2-targeting vector was successfully obtained and included a complete sequence as shown by SEQ ID NO: 27.

### Example 5

A continuous fragment 5arm-hACE-SV40 was obtained from the PCR amplification products, i.e., the 5' homology arm, the human ACE2 CDS, and the SV40 polyA by means of the overlap PCR. The PCR conditions are shown in Table 5, and the remaining conditions are the same as those in Example 4.

**Table 5. PCR system (50 µL)**

| | |
|---|---|
| 2×Phanta Max Buffer | 25 µL |
| dNTP Mix | 1 µL |
| Upstream primer (10µM) | 2 µL |
| Downstream primer (10µM) | 2 µL |
| Phanta Max Super-Fidelity DNA Polymerase | 1 µL |
| Templates | 5' homology arm, human ACE2 CDS, and SV40 polyA fragments each 50 ng |
| H₂O | Up to 50 µL |
| PCR amplification reaction conditions | 65°C |

The results are shown in Fig. 8. It shows the continuous fragment comprising the above three fragments was failed to be amplified.

### Example 6. Obtaining of mouse embryonic stem cells having humanized ACE2

In one embodiment, the mouse embryonic stem cells having humanized ACE2 were obtained by the following process.
(1) C57BL6/J mouse embryonic stem cells, which were derived from an established embryonic stem cell line, were revived from liquid-nitrogen cryopreserved cells. The mouse embryonic stem cells within passage 10 (p10) were particularly used and cultured in a 6-cm dish for 3 days.
(2) About 2×10⁶ embryonic stem cells were electroporated by using Nucleofector^{™} IIs/2b electroporation device under A023 program, in conjunction with a Mouse ES Cell Nucleofector^{®} Kit (Lonza,VPH-1001). The electroporation was performed in 100 µL of an electroporation buffer containing 3 µg of the linearized targeting vector which was prepared in Example 4, and 1 µg of pX330-sgRNA1. The transfected cells were seeded in three 6-well plates and cultured for 36 hours. After that, 1 µg/mL of puromycin (Merck & Co.) was added into the cell culture medium.

After 3-4 days of screening, the puromycin-resistant mouse embryonic stem cell clones were picked by means of sucking monoclones with a glass needle, and placed in 96-well plates for culturing. The following day, the monoclones were subjected trypsinization and divided into two parts of cells. One part of the cells was lysed in 10 µL of NP 40 lysis buffer at 56°C for 60 min, following by lysing at 95°C for 10 min.

The other part of the mouse embryonic stem cells was cultured as follows. Feeder cells were prepared one day in advance, and spread in different well-plates as required and cultured overnight to form a single layer. In particular, the feeder cells were mitomycin C (MMC)-treated mouse embryonic fibroblasts. The feeder cells were cultured in mES medium containing LIF and 2i (chir99021- and pD0325901-inhibitors). The medium was replaced every day with fresh medium, and the amount of the medium could be increased appropriately depending on the growth of the cells. The cells were usually passaged every 3 days. For the passage, the cells were digested with 0.25% trypsin, and then seeded at a density of about 300,000 in a 6-cm plate and about 100,000 per well in a 6-well plate.

The mES+LIF+2i medium contained Knockout DMEM (1×, gibco) + 15% FBS (FRONT BIOMEDICAL, 0.22 µm Millipore filter-filtered) + GlutaMAX (100×, gibco) + NEAA (100×, gibco) + P/S (P: 50 units, S: 50 mg/ml, Hyclone) + β-mercaptoethanol (gibco, working concentration of 0.1 mM) + LIF (1000 units/ml, Millipore) + CHIR99021 (GSK3β inhibitor, working concentration of 3µM) + PD0325901 (MEK inhibitor, working concentration of 1 µM).

The NP40 lysis buffer was composed of 10 mL TE (20 mM Tris pH8.0, 150 mM NaCl, 2 mM EDTA) + 0.5% NP40 + 10 µL proteinase K (10 mg/mL).

The pX330-sgRNA1 used in the above process was replaced with pX330-sgRNA2 or pX330-sgRNA3. Remaining steps were the same as those for the pX330-sgRNA1.

The targeting effects of pX330-sgRNA1, pX330-sgRNA2 and pX330-sgRNA3 in combination with the targeting vector are shown in Table 6.

**Table 6. Targeting effects (number of surviving clones) of sgRNA1-3 in combination with the targeting vector.**

| pX330-sgRNA1 | pX330-sgRNA2 | pX330-sgRNA3 |
|---|---|---|
| 231 | 187 | 43 |

### Example 7. Genotype identification of mouse embryonic stem cells having humanized ACE2

The cell lysate obtained from Example 6 was used as the template for the genotype PCR screen. The PCR screen was performed by using Phanta Max Super-Fidelity DNA Polymerase reagent (Novizan) following the manufacturer's instructions. PCR was used for analyzing HDR having 5' and 3' homology arms, which was directionally inserted into the mouse ACE2 site. No biallelic targeting could occur because the ACE2 gene was located on the X chromosome and the mouse embryonic stem cells were derived from XY males.

For the identification of the directed insertion, the upstream primer was located inside the puromycin-resistance gene (Puro-F: aacctccccttctacgagc (SEQ ID NO: 28)), and the matching downstream primer was located downstream of the 3' homology arm (3arm-outR: tacagccaggatctggatgtcagc (SEQ ID NO: 29)). If the recombinant vector was inserted correctly, a 1504bp band should appear. In such case, the ACE2 genomic sequence of the mouse embryonic stem cells was replaced with SEQ ID NO: 30.

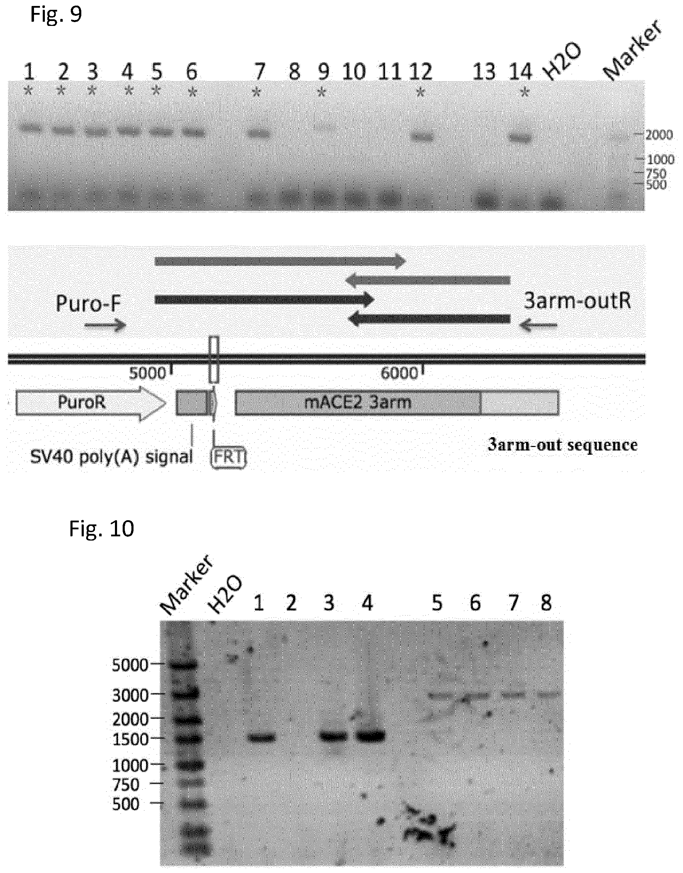

The PCR identification results are shown in Fig. 9. A total of 14 clones were identified, in which the clones marked with "*" were positive clones and were also consistent with the sequencing results of the PCR products. The mouse embryonic stem cell clones, which had positive PCR results, were successfully edited and humanized mouse embryonic stem cell models. The PGK-Puro selection marker was still present in these models, and was flanked with a pair of frt sites which were oriented in the same direction. Thus, the PGK-Puro could be removed by using FLP recombinase, thereby solving the safety issues caused by transgenosis.

The PGK-Puro selection marker was removed by the following process.

The mouse embryonic stem cells, which had positive PCR results, were further cultured in 6 cm-plates. 2×10⁶ cells were electroporated by using a Nucleofector^{™} IIs/2b electroporation device under A023 program, in conjunction with a Mouse ES Cell Nucleofector^{®} Kit (Lonza, VPH-1001). The electroporation was performed in 100 µL of an electroporation buffer containing pPGK-FLPo plasmid (Addgene, 13793). The transfected cells were seeded into six 12-well plates. After 3 days, the mouse embryonic stem cells were picked by means of sucking monoclones with a glass needle, and placed in 96-well plates for culturing. The following day, the monoclones were passaged by trypsinization and divided into two parts of cells, one part for puromycin selection and the other part for normal culture. If the PGK-Puro resistance selection marker was successfully removed, the cells would die due to puromycin intolerance. The puromycin-intolerant clones were subjected to genotype PCR identification by using an upstream primer (hACE2-F: tgatagtggttggcattgt (SEQ ID NO:31)) and a downstream primer (3arm-outR: tacagccaggatctggatgtcagc (SEQ ID NO: 29)). The PCR products had a length of 2863 bp in the presence of PGK-Puro. The PCR products would have a length of 1532 bp when the PGK-Puro selection marker was successfully removed. The genotype identification results are shown in Fig. 9. As shown in Fig. 9, the clones with 1500bp bands were clones in which the PGK-puro had been successfully removed, and the clones with 3000bp bands were clones in which the PGK-puro remained. The puromycin-intolerant clones were the mouse embryonic stem cells which had been successfully edited and had human ACE2. That is, the mouse embryonic stem cells finally obtained the humanized ACE2 gene as shown in Fig. 10. After the removal of the PGK-puro, the ACE2 genomic sequence of the mouse embryonic stem cells was replaced with the sequence as shown by SEQ ID NO: 32.

### Example 8. Formation of humanized ACE2 gene-engineered mice

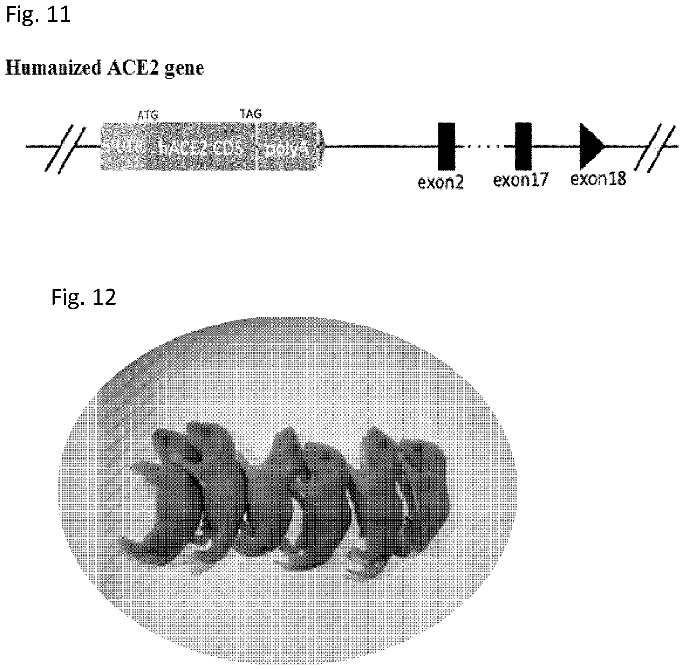

1. Each of 4-10 week old B6C3F1 female mice was intraperitoneally injected with 7.5 units of PMSG. After 48 hours, each of the female mice was injected with hCG and co-caged with CD1 male mice. On the next morning, the female mice were checked for vaginal plugs and those with vaginal plugs were picked out. The fertilization time of each of the female mice was recorded.
2. On the next day, each of the pregnant mice was euthanized by cervical dislocation, and was sterilized with 70% alcohol for the abdomen thereof. Then, the abdominal skin and muscle layers were cut with forceps and ophthalmic scissors to expose the abdominal cavity. With the upper part of the uterine horn being held by the forceps, a small slit was cut with the scissors in the membrane near the fallopian tube, and the junction between the fallopian tube and the ovary was cut. The fallopian tube and the attached uterus were transferred to a 35-mm culture dish. The fimbria of the fallopian tube was fixed with the forceps. Then, a flushing needle filled with M2 medium (Hogan, B. (1994). Manipulating the mouse embryo: a laboratory manual, 2nd edn (Cold Spring Harbor, NY, Cold Spring Harbor Laboratory Press)) was gently inserted into the fimbria to flush the fallopian tube with 0.1 mL of M2 medium. The embryos were flushed out, collected with an ovum transfer tube and washed three times with M2. E1.5 mouse embryos at 2-cell stage were collected.
3. The collected mouse embryos were placed in 0.3 M mannitol (Sigma-Aldrich Inc., St.Louis, MO) containing 0.1 mM MgSO₄, 0.1 mM CaCl₂, and 0.3% bovine serum albumin, and subjected to direct current fusion at 60 V for 50 microseconds by using Cellfusion CF-150/B electrofusion device and a 250-µm fusion tank (BLS Ltd., Budapest, Hungary), to obtain tetraploid embryos. The tetraploid embryos were then put into KSOM medium (Summers, M.C., McGinnis, L.K., Lawitts, J.A., Raffin, M., and Biggers, J.D. (2000). IVF of mouse ova in a simplex optimized medium supplemented with amino acids. Hum Reprod 15, 1791-1801). After culturing in a CO₂ incubator for 24 hours, the zona pellucida of each of the embryos was removed by treatment with acidic Tyrode's solution (Sigma-Aldrich, T1788). Then the embryos were aggregated with the embryonic stem cells (the mouse embryonic stem cells having humanized ACE2 obtained in Example 6) to form chimeric embryos (Nagy, A., Rossant, J., Nagy, R., Abramow-Newerly, W, and Roder, J.C. (1993). Derivation of completely cell culture-derived mice from early-passage embryonic stem cells. Proc Natl Acad Sci US A 90, 8424-8428.).
4. The chimeric embryos were cultured overnight in a CO₂ incubator, and then transplanted into the uterus of a pseudopregnant E2.5 mouse. 17 days later, the surrogate mouse was euthanized by cervical dislocation and subjected to a cesarean section. The surviving and breathing neonatal mice were placed in a cage having a lactating mouse. The neonatal mice were weaned 21 days later, and then were transgenic mice derived from the embryonic stem cells, i.e., humanized ACE2 gene-engineered mice (Fig. 12).

The corresponding culture media contained the following components as shown in Table 7.

**Table 7. Components of culture media**

| Culture media | KSOM (200 mL) | M2 (200 mL) |
|---|---|---|
| EDTA (disodium 0.01 mM) | 0.00076 g | / |
| NaCl | 1.119 g | 1.1068 g |
| KCl | 0.037 g | 0.070 g |
| CaCl2·2H2O | 1.71 mM | 1.71 mM |
| KH2PO4 | 0.0095 g | 0.032 g |
| MgSO₄ | 0.00482 g | 0.0283 g |
| NaHCO3 | 0.420 g | 0.070 g |
| Na lactate | 0.280 mL | 0.62 mL |
| Na pyruvate | 0.0044 g | 0.0073 g |
| Glucose | 0.0072 g | 0.1 g |
| Pen/Strep/Glu | 1 mL | 2 mL |
| HEPES | / | 0.994 g |
| EDTA (100 mM) | / | 200 µL |
| Gentamycin | / | 200 µL |
| Phenol red (0.5%) | 20 µL | 100 µL |
| BSA | 0.2 g | 0.8 g |
| Water | Up to 200 mL | Up to 200 mL |

### Example 9. Identification of expression in humanized ACE2 mice

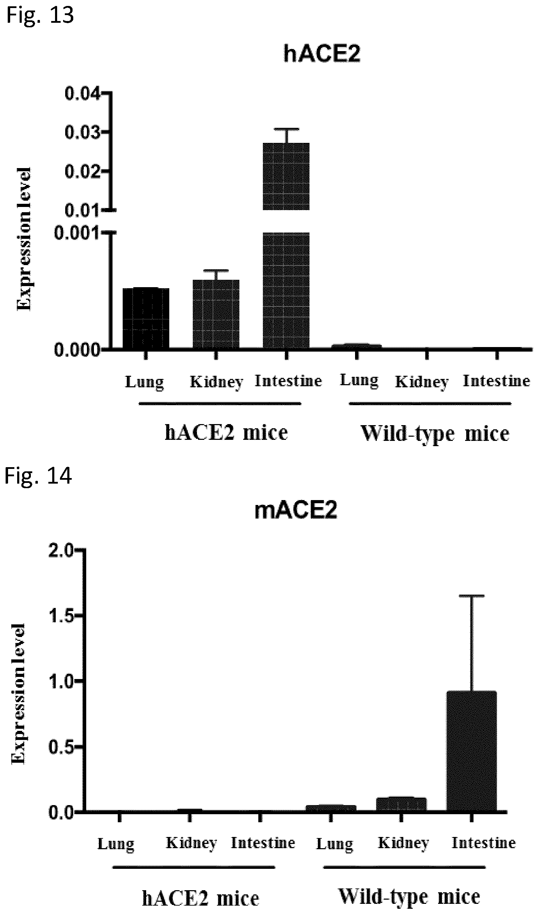

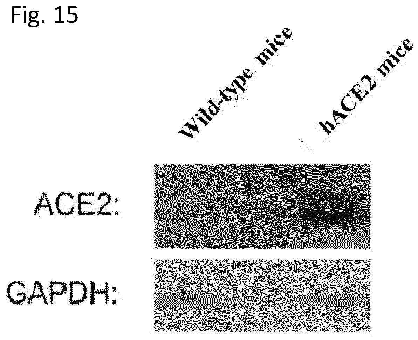

Neonatal humanized ACE2 mice and wild-type mice were dissected. Lungs, kidneys, and intestines of these mice were taken out and lysed respectively with Trizol. RNA was then extracted with a cell/tissue total RNA extraction kit (Novezan, RC101-01) for reverse transcription with HiScript II Q RT SuperMix for qPCR reagent (Novizan, R222-01). cDNA extracted from the tissues was detected by qPCR using hACE2-specific primers (hACE2-qF: TGATAGTGGTTGGCATTGT, hACE2-qR: CGATGGAGGCATAAGGATT) and mACE2-specific primers (mACE2-qF: GGTTGGCATCATCCT; mACE2-qR: GTCTGAGCATCATCACTGT) (Novizant, Q321-02)). The results show that the tissues of the humanized ACE2 mice specifically expressed hACE2, whereas the wild-type mice did not express hACE2 (Fig. 13), while mACE2 expression was missing in the hACE2 mice (Fig. 14). Further, the intestinal tissues of the hACE2 mice and the wild-type ACE2 mice were taken for immunoblotting assay. The sodium dodecyl sulfate polyacrylamide gel electrophoresis (SDS-PAGE) was performed using a human-specific ACE2 antibody (Abeam, ab108209) to confirm that hACE2 mice expressed hACE2 protein at the protein level (Fig. 15).

### Example 10. Method for preparation of mice (2-cell stage/electrofusion solution with increased Mg²⁺ and Ca²⁺ concentrations)

The mouse embryonic stem cells used in this example were the puromycin-intolerant human ACE2-targeted mouse embryonic stem cells obtained in Examples 6 and 7. In a specific experiment, such cells were revived from liquid-nitrogen cryopreserved cells. The embryonic stem cells within passage 15 (p15) were particularly used, and cultured in a 6-cm dish for 3 days. The cells used in this example were embryonic stem cells at passage 12.
1. The solutions used in Example 10 contained the components as shown in Tables 8-10.

**Table 8. Components of the electrofusion solution (with increased Mg²⁺ and Ca²⁺ concentrations)**

| **Components** | **Contents** |
|---|---|
| Mannitol | 0.27 M |
| MgSO₄ | 0.2 mM |
| CaCl₂ | 0.2 mM |
| Bovine Serum Albumin | 3 mg/mL |
| The balance is water. | |

**Table 9. Components of the culture media**

| **Culture medium** | **KSOM (200 mL)** | **M2 (200 mL)** |
|---|---|---|
| EDTA (disodium 0.01 mM) | 0.00076 g | / |
| NaCl | 1.119 g | 1.1068 g |
| KCl | 0.037 g | 0.070 g |
| CaCl₂ . 2H₂O | 1.71 mM | 1.71 mM |
| KH₂PO₄ | 0.0095 g | 0.032 g |
| MgSO₄ | 0.00482 g | 0.0283 g |
| NaHCO₃ | 0.420 g | 0.070 g |
| Na lactate | 0.280 mL | 0.62 mL |
| Na pyruvate | 0.0044 g | 0.0073 g |
| Glucose | 0.0072 g | 0.1 g |
| Pen/Strep/Glu | 1 mL | 2 mL |
| HEPES | / | 0.994 g |
| EDTA (100mM) | / | 200 µL |
| Gentamycin | / | 200 µL |
| Phenol red (0.5%) | 20 µL | 100 µL |
| BSA | 0.2 g | 0.8 g |
| water | Up to 200mL | Up to 200mL |

**Table 10. Acidic Tyrode's solution**

| **Components** | **Contents (g/100 mL)** |
|---|---|
| NaCl | 0.800 |
| KCl | 0.020 |
| CaCl₂•2H₂O | 0.024 |
| MgSO₄ | 0.010 |
| Glucose | 0.100 |
| PVP (Polyvinylpyrrolidone) | 0.400 |
| Adjust to pH 2.5 with hydrochloric acid, and store at -20°C in aliquots. Also available from Sigma-Aldrich (T1788) | |

2. Preparation of mice
(1) Each of 4-10 week-old B6C3F1 female mice was intraperitoneally injected with 7.5 units of PMSG. After 48 hours, each of the female mice was injected with hCG and co-caged with CD1 male mice. On the next morning, the female mice were checked for vaginal plugs and those with vaginal plugs were picked out. The fertilization time of each of the female mice was recorded.
(2) On the next day, each of the pregnant mice was euthanized by cervical dislocation, and their abdomens were sterilized with 70% alcohol. Then, the abdominal skin and muscle layers were cut with forceps and ophthalmic scissors to expose the abdominal cavity. With the upper part of the uterine horn being held by the forceps, a small slit was cut with the scissors in a membrane near the fallopian tube, and the junction between the fallopian tube and the ovary was cut. The fallopian tube and the attached uterus were transferred to a 35-mm culture dish. The fimbria of the fallopian tube was fixed with the forceps. Then, a flushing needle filled with M2 medium (Hogan, 1994) was gently inserted into the fimbria to flush the fallopian tube with 0.1 mL of M2 medium. The embryos were flushed out, collected with an ovum transfer tube, and washed three times with M2. Then E1.5 (1.5 days after fertilization) mouse embryos at 2-cell stage were collected.
(3) The collected mouse 2-cell embryos were placed in an improved electrofusion solution (with the components listed in Table 8), and subjected to direct current fusion at 60 volts for 50 microseconds using a Cellfusion CF-150/B electrofusion device and a fusion tank with electrodes spaced 250-microns apart (BLS Ltd., Budapest, Hungary) to obtain tetraploid embryos. Then, the fused embryos were put into KSOM medium (Summers et al., 2000), and cultured in a CO₂ incubator for 15 hours. The results showed that the improved electrofusion solution enabled the fusion efficiency to reach 100%.
(4) As the tetraploid embryos developed to 2-cell stage, the zona pellucida of each of the embryos was removed by treatment with acidic Tyrode's solution. Then the embryos were aggregated with small clusters of embryonic stem cells digested with 0.25% trypsin, and cultured for another 24 hours to form chimeric embryos.
(5) Finally, the chimeric embryos were transplanted into the uterus of a pseudopregnant E2.5 mouse. 17 days later, the full-term neonatal mice were obtained.

Since the tetraploid embryos could only effectively form extraembryonic tissues such as placenta, the neonatal mice, which were obtained from the chimera embryos of the tetraploid embryos and the embryonic stem cells, were fully derived from the embryonic stem cells.

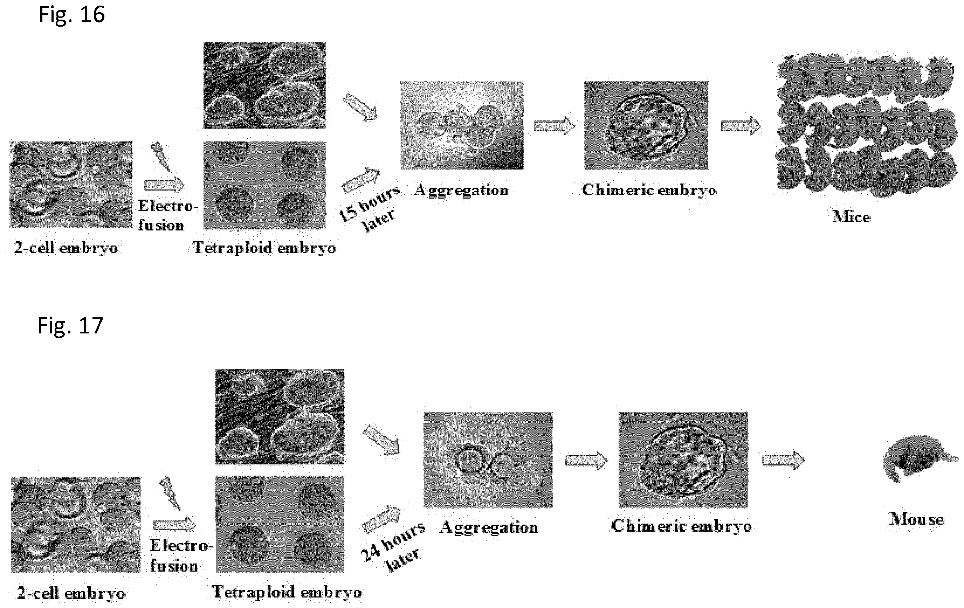

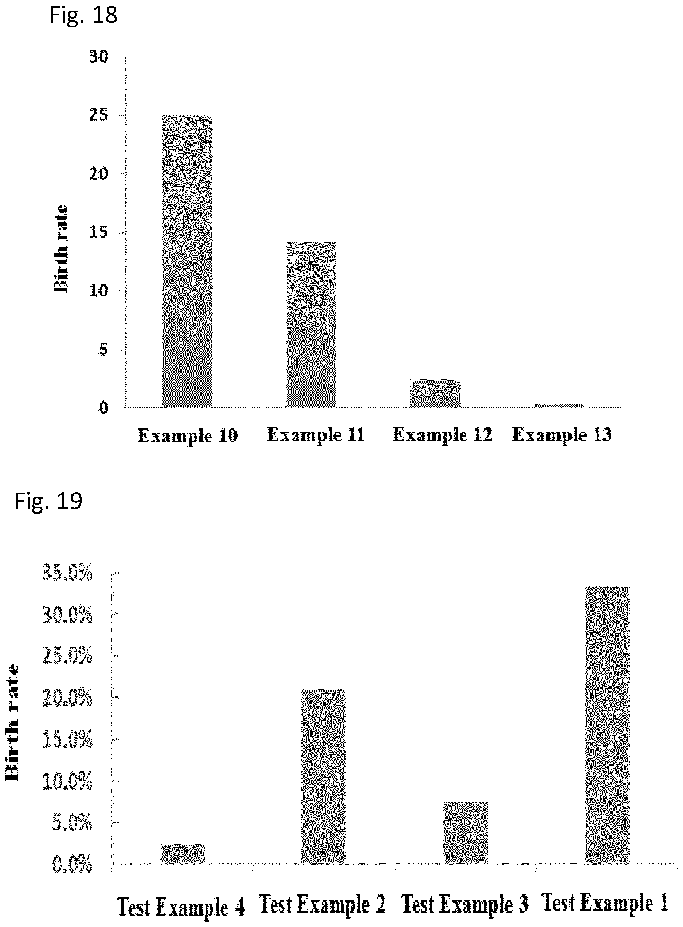

The schematic flow chart of the preparation of mice in this example is shown in Fig. 16. The results of the birth rate of the mice are shown in Fig. 18. As can be seen from Fig. 18, the survival rate of the mice prepared by the method of Example 10 reaches 25% (10/40).

### Test Example 1

The steps in this test example were the same as those in Example 10, except that the step of obtaining the embryonic stem cells was different from that in Example 10.

In this test example, the mouse embryonic stem cells were ordinary and non-engineered embryonic stem cells, which were obtained as follows. C57BL6/J mouse embryonic stem cells were revived from liquid-nitrogen cryopreserved cells. The mouse embryonic stem cells at passage 12 were used, and cultured in a 6-cm dish for 3 days.

The schematic diagram of the process for preparing mice is shown in Fig. 16. The results of the birth rate of the mice are shown in Fig. 19. As can be seen from Fig. 19, the survival rate of the mice prepared by the method of Test Example 1 reaches 33.3% (48/144).

### Example 11. Preparation method of mice (2-cell stage/traditional fusion solution)

The mouse embryonic stem cells used in this example were the puromycin-intolerant human ACE2-targeted mouse embryonic stem cells obtained in Examples 6 and 7. In a specific experiment, such cells were revived from liquid-nitrogen cryopreserved cells. The embryonic stem cells within passage 15 (p15) were particularly used, and cultured in a 6-cm dish for 3 days. The cells used in this example were embryonic stem cells at passage 12.

The used KSOM medium and M2 medium were the same as those used in Example 10.
1. Each of 4-10 week-old B6C3F1 female mice was intraperitoneally injected with 7.5 units of PMSG. After 48 hours, each of the female mice was injected with hCG and co-caged with CD1 male mice. On the next morning, the female mice were checked for vaginal plugs and those with vaginal plugs were picked out. The fertilization time of each of the female mice was recorded.
2. On the next day, each of the pregnant mice was euthanized by cervical dislocation, and was sterilized with 70% alcohol for the abdomen thereof. Then, the abdominal skin and muscle layers were cut with auxiliary forceps and ophthalmic scissors to expose the abdominal cavity. With the upper part of the uterine horn being held by the forceps, a small slit was cut with the scissors in a membrane near the fallopian tube, and the junction between the fallopian tube and the ovary was cut. The fallopian tube and the attached uterus were transferred to a 35-mm culture dish. The fimbria of the fallopian tube was fixed with the forceps. Then, a flushing needle filled with M2 medium (Hogan, 1994) was gently inserted into the fimbria to flush the fallopian tube with 0.1 mL of M2 medium. The embryos were flushed out, collected with an ovum transfer tube, and washed three times with M2. Then E1.5 mouse embryos at 2-cell stage were collected.
3. The collected mouse 2-cell embryos were placed in an electrofusion solution (with the components listed in Table 11), and subjected to direct current fusion at 60 volts for 50 microseconds using Cellfusion CF-150/B electrofusion device and a fusion tank with electrodes spaced 250-micron apart (BLS Ltd., Budapest, Hungary) to obtain tetraploid embryos. Then, the fused embryos were put into KSOM medium (Summers et al., 2000), and cultured in a CO₂ incubator for 24 hours. The results showed that the fusion efficiency is 80-90%, which is less than 100%.
4. As the tetraploid embryos were developed to 2-cell stage, the zona pellucida of each of the embryos was removed by treatment with acidic Tyrode's solution. Then the embryos were aggregated with small clusters of embryonic stem cells digested with 0.25% trypsin, and cultured for another 24 hours to form chimeric embryos. Alternatively, the tetraploid embryos were cultured for 48 hours to the blastocyst stage, and then the embryonic stem cells were micro-injected into the blastocysts (Nagy et al., 1993).
5. Finally, the chimeric embryos were transplanted into the uterus of a pseudopregnant E2.5 mouse. 17 days later, the full-term neonatal mice were obtained. Since the tetraploid embryos could only effectively form extraembryonic tissues such as placenta, the neonatal mice, which were obtained from the the chimera embryos of the tetraploid embryos and the embryonic stem cells, were fully derived from the embryonic stem cells.

**Table 11. Components of the electrofusion solution (traditional fusion solution).**

| **Components** | **Contents** |
|---|---|
| Mannitol | 0.3 M |
| MgSO₄ | 0.1 mM |
| CaCl₂ | 0.1 mM |
| Bovine Serum Albumin | 3 mg/mL |
| The balance is water | |

The schematic diagram of the process for preparing the mice is shown in Fig. 16. The results of the birth rate of the mice in this example are shown in Fig. 18. As can be seen from Fig. 18, the survival rate of the mice is 14.2% (15/106).

### Test Example 2

The steps in this test example were the same as those in Example 11, except that the step of obtaining of the embryonic stem cells was different from that in Example 11.

In this test example, the mouse embryonic stem cells were ordinary and non-engineered embryonic stem cells, which were obtained as follows. C57BL6/J mouse embryonic stem cells were revived from liquid-nitrogen cryopreserved cells. The mouse embryonic stem cells at passage 12 were used and cultured in a 6-cm dish for 3 days.

The schematic diagram of the process for preparing mice is shown in Fig. 16. The results of the birth rate of the mice are shown in Fig. 19. As can be seen from Fig. 19, the survival rate of the mice is 21.1% (32/152).

### Example 12 Preparation method of mice (4-cell stage/electrofusion solution with increased Mg²⁺ and Ca²⁺ concentrations)

The mouse embryonic stem cells used in this example were the puromycin-intolerant human ACE2-targeted mouse embryonic stem cells obtained in Examples 6 and 7. In a specific experiment, such cells were revived from liquid-nitrogen cryopreserved cells. The embryonic stem cells within passage 15 (p15) were particularly used, and then cultured in a 6-cm dish for 3 days. The cells used in this example were embryonic stem cells at passage 12.

The used KSOM medium and M2 medium were the same as those used in Example 10.
1. Each of 4-10 week-old B6C3F1 female mice was intraperitoneally injected with 7.5 units of PMSG. After 48 hours, each of the female mice was injected with hCG and co-caged with CD1 male mice. On the next morning, the female mice were checked for vaginal plugs and those with vaginal plugs were picked out. The fertilization time of each of the female mice was recorded.
2. On the next day, each of the pregnant mice was euthanized by cervical dislocation, and was sterilized with 70% alcohol for the abdomen thereof. Then, the abdominal skin and muscle layers were cut with auxiliary forceps and ophthalmic scissors to expose the abdominal cavity. With the upper part of the uterine horn being held by the forceps, a small slit was cut with the scissors in a membrane near the fallopian tube, and the junction between the fallopian tube and the ovary was cut. The fallopian tube and the attached uterus were transferred to a 35-mm culture dish. The fimbria of the fallopian tube was fixed with the forceps. Then, a flushing needle filled with M2 medium (Hogan, 1994) was gently inserted into the fimbria to flush the fallopian tube with 0.1 mL of M2 medium. The embryos were flushed out, collected with an ovum transfer tube, and washed three times with M2. Then E1.5 mouse embryos at 2-cell stage were collected.
3. The collected mouse 2-cell embryos were placed in an electrofusion solution (with the components listed in Table 12), and subjected to direct current fusion at 60 volts for 50 microseconds using Cellfusion CF-150/B electrofusion device and a fusion tank with electrodes spaced 250-micron apart (BLS Ltd., Budapest, Hungary) to obtain tetraploid embryos. Then, the fused embryos were put into KSOM medium (Summers et al., 2000), and cultured in a CO₂ incubator for 24 hours. The results showed that the improved electrofusion solution enabled the fusion efficiency to reach 100%.
4. As the tetraploid embryos were developed to 4-cell stage, the zona pellucida of each of the embryos was removed by treatment with acidic Tyrode's solution. Then the embryos were aggregated with small clusters of embryonic stem cells digested with 0.25% trypsin, and cultured for another 24 hours to form chimeric embryos. Alternatively, the tetraploid embryos were cultured for 48 hours to the blastocyst stage, and then the embryonic stem cells were micro-injected into the blastocysts (Nagy et al., 1993).
5. Finally, the chimeric embryos were transplanted into the uterus of a pseudopregnant E2.5 mouse. 17 days later, the full-term neonatal mice were obtained. Since the tetraploid embryos could only effectively form extraembryonic tissues such as placenta, the neonatal mice, which were obtained from the chimera embryos of the tetraploid embryos and the embryonic stem cells, were fully derived from the embryonic stem cells.

**Table 12. Components of the electrofusion solution (with increased Mg²⁺ and Ca²⁺ concentrations)**

| **Components** | **Contents** |
|---|---|
| Mannitol | 0.27 M |
| MgSO₄ | 0.2 mM |
| CaCl₂ | 0.2 mM |
| Bovine Serum Albumin | 3 mg/mL |
| The balance is water | |

The schematic diagram of the process for preparing the mice is shown in Fig. 17.

The results of the birth rate of the mice in this example are shown in Fig. 18. As can be seen from Fig. 18, the survival rate of the mice is 2.5% (3/121).

### Test Example 3

The steps in this test example were the same as those in Example 12, except that the step of obtaining of the embryonic stem cells was different from that in Example 12.

In this test example, the mouse embryonic stem cells were ordinary and non-engineered embryonic stem cells, which were obtained as follows. C57BL6/J mouse embryonic stem cells were revived from liquid-nitrogen cryopreserved cells. The mouse embryonic stem cells at passage 12 were used, and cultured in a 6-cm dish for 3 days.

The schematic diagram of the process for preparing mice is shown in Fig. 17. The results of the birth rate of the mice are shown in Fig. 19. As can be seen from Fig. 19, the survival rate of the mice is 7.5% (9/120).

### Example 13. Preparation method of mice (4-cell stage/traditional fusion solution)

The mouse embryonic stem cells used in this example were the puromycin-intolerant human ACE2-targeted mouse embryonic stem cells obtained in Examples 6 and 7. In a specific experiment, such cells were revived from liquid-nitrogen cryopreserved cells. The embryonic stem cells within passage 15 (p15) were particularly used, and then cultured in a 6-cm dish for 3 days. The cells used in this example were embryonic stem cells at passage 12. The used KSOM medium and M2 medium were the same as those used in Example 10.
1. Each of 4-10 week-old B6C3F1 female mice was intraperitoneally injected with 7.5 units of PMSG. After 48 hours, each of the female mice was injected with hCG and co-caged with CD1 male mice. On the next morning, the female mice were checked for vaginal plugs and those with vaginal plugs were picked out. The fertilization time of each of the female mice was recorded.
2. On the next day, each of the pregnant mice was euthanized by cervical dislocation, and was sterilized with 70% alcohol for the abdomen thereof. Then, the abdominal skin and muscle layers were cut with auxiliary forceps and ophthalmic scissors to expose the abdominal cavity. With the upper part of the uterine horn being held by the forceps, a small slit was cut with the scissors in a membrane near the fallopian tube, and the junction between the fallopian tube and the ovary was cut. The fallopian tube and the attached uterus were transferred to a 35-mm culture dish. The fimbria of the fallopian tube was fixed with the forceps. Then, a flushing needle filled with M2 medium (Hogan, 1994) was gently inserted into the fimbria to flush the fallopian tube with 0.1 mL of M2 medium. The embryos were flushed out , collected with an ovum transfer tube, and washed three times with M2. Then E1.5 mouse embryos at 2-cell stage were collected.
3. The collected mouse 2-cell embryos were placed in an electrofusion solution (with the components listed in Table 13), and subjected to direct current fusion at 60 volts for 50 microseconds using Cellfusion CF-150/B electrofusion device and a fusion tank with electrodes spaced 250-micron apart (BLS Ltd. , Budapest, Hungary) to obtain tetraploid embryos. Then, the fused embryos were put into KSOM medium (Summers et al., 2000), and cultured in a CO2 incubator for 24 hours. The electrofusion efficiency was 80-90%.
4. As the tetraploid embryos were developed to 4-cell stage, the zona pellucida of each of the embryos was removed by treatment with acidic Tyrode's solution. Then, the embryos were aggregated with small clusters of embryonic stem cells digested with 0.25% trypsin, and cultured for another 24 hours to form chimeric embryos. Alternatively, the tetraploid embryos were cultured for 48 hours to the blastocyst stage, and then the embryonic stem cells were micro-injected into the blastocysts (Nagy et al., 1993)
5. Finally, the chimeric embryos were transplanted into the uterus of a pseudopregnant E2.5 mouse. 17 days later, the full-term neonatal mice were obtained. Since the tetraploid embryos can only effectively form extraembryonic tissues such as placenta, the neonatal mice, which were obtained from the chimera embryos of the tetraploid embryos and the embryonic stem cells, were fully derived from the embryonic stem cells.

**Table 13. Components of the electrofusion solution (traditional fusion solution)**

| **Components** | **Contents** |
|---|---|
| Mannitol | 0.3 M |
| MgSO₄ | 0.1 mM |
| CaCl₂ | 0.1 mM |
| Bovine Serum Albumin | 3 mg/mL |
| The balance is water. | |

The process and results of preparing the mice in this example 5 are shown in Fig. 17. As can be seen from Fig. 17, the mouse preparation efficiency is very low. Further, the results of the birth rate of the mice are shown in Fig. 18. As can be seen from Fig. 18, the survival rate of the mice is only 0.3% (1/350).

### Test Example 4

The steps in this test example were the same as those in Example 13, except that the step of obtaining of the embryonic stem cells was different from that in Example 13.

In this test example, the mouse embryonic stem cells were ordinary and non-engineered embryonic stem cells, which were obtained as follows. C57BL6/J mouse embryonic stem cells were revived from liquid-nitrogen cryopreserved cells. The mouse embryonic stem cells at passage 12 were used and cultured in a 6-cm dish for 3 days.

The schematic diagram of the process for preparing mice is shown in Fig. 17. The results of the birth rate of the mice are shown in Fig. 19. As can be seen from Fig. 19, the survival rate of the mice is only 2.5% (4/163).

## Claims

1. A method for preparing a chimeric embryo, comprising aggregating a tetraploid embryo with embryonic stem cells to form a new reconstructed embryo or a chimeric embryo, wherein the tetraploid embryo is a tetraploid embryo at 2-cell stage;
preferably, the method adopts a tetraploid complementation assay or a tetraploid embryo complementation assay;
preferably, the method comprises the following steps:
(1) obtaining an animal 2-cell embryo;
(2) placing the 2-cell embryo obtained in step (1) in a fusion solution and performing fusion to obtain a tetraploid embryo;
(3) placing the tetraploid embryo obtained in step (2) in a culture medium and culturing;
(4) aggregating the tetraploid embryo, which is developed to 2-cell stage in step (3), with embryonic stem cells to form a chimeric embryo;
preferably, in step (3), the tetraploid embryo is cultured for about 8-24 hours;
or preferably, the culture medium in step (3) is KSOM medium.

2. A method for preparing a non-human animal, comprising aggregating a tetraploid embryo with embryonic stem cells to form a new reconstructed embryo or a chimeric embryo, wherein the tetraploid embryo is a tetraploid embryo at 2-cell stage;
preferably, the method adopts a tetraploid complementation assay or a tetraploid embryo complementation assay;
preferably, the method comprises the following steps:
(1) obtaining an animal 2-cell embryo;
(2) placing the 2-cell embryo obtained in step (1) in a fusion solution and performing fusion to obtain a tetraploid embryo;
(3) placing the tetraploid embryo obtained in step (2) in a culture medium and culturing;
(4) aggregating the tetraploid embryo, which is developed to 2-cell stage in step (3), with embryonic stem cells to form a chimeric embryo;
preferably, in step (3), the tetraploid embryo is cultured for about 8-24 hours;
or preferably, the culture medium in step (3) is KSOM medium.

3. The method according to claim 1 or 2, wherein the animal is a mammal;
preferably, the animal is a non-human mammal;
preferably, the animal is selected from a group consisting of pig, rat, mouse, hamster, rabbit, pig, bovine, deer, sheep, goat, chicken, cat, horse, dog, orangutan, and monkey;
preferably, the animal is selected from a murine;
preferably, the animal is selected from an adult murine;
preferably, the animal is selected from a fetal murine;
preferably, the animal is a gene-edited animal;
preferably, the animal is an animal with a humanized gene;
preferably, the gene is ACE2.

4. The method according to claim 1 or 2, wherein the fusion employs a composition comprising mannitol, MgSO₄, CaCl₂, and bovine serum albumin in a mass ratio of 9-53 : 0.015-0.241 : 0.013-0.23 : 0.01-5;
preferably, the composition comprises mannitol, MgSO₄, CaCl₂ and bovine serum albumin in a mass ratio of 18-52 : 0.018-0.241 : 0.016-0.23 : 0.01-4;
preferably, the composition comprises mannitol, MgSO₄, CaCl₂ and bovine serum albumin in a mass ratio of 27-52 : 0.018-0.12 : 0.016-0.1 : 1-3.5;
preferably, the composition comprises 0.05-0.29 M of mannitol, 0.12-2 mM of MgSO₄, 0.12-2 mM of CaCl₂, and 0.01-5 mg/mL of bovine serum albumin;
preferably, the composition comprises 0.1-0.28 M of mannitol, 0.15-2 mM of MgSO₄, 0.15-2 mM of CaCl₂, and 0.01-4 mg/mL of bovine serum albumin;
preferably, the composition comprises 0.15-0.28 M of mannitol, 0.15-1 mM of MgSO₄, 0.15-1 mM of CaCl₂, and 1-3.5 mg/mL of bovine serum albumin;
more preferably, the composition comprises 0.18-0.28 M of mannitol, 0.15-0.5 mM of MgSO₄, 0.15-0.5 mM of CaCl₂, and 2-3.5 mg/mL of bovine serum albumin;
more preferably, the composition comprises 0.2-0.28 M of mannitol, 0.15-0.3 mM of MgSO₄, 0.15-0.3 mM of CaCl₂, and 2-3 mg/mL of bovine serum albumin;
preferably, the composition is an electrofusion solution;
or preferably, the electrofusion solution comprises mannitol, bovine serum albumin, Mg²⁺ and Ca²⁺, and the electrofusion solution comprises increased concentrations of Mg²⁺ and Ca²⁺; or, the electrofusion solution comprises 0.12-2 mM of Mg²⁺ and 0.12-2 mM of Ca²⁺;
preferably, the electrofusion solution comprises 0.05-0.29 M of mannitol and 0.01-5 mg/mL of bovine serum albumin;
more preferably, Mg²⁺ and Ca²⁺ are derived from MgSO₄ and CaCl₂, respectively;
more preferably, the electrofusion solution is used for preparing the tetraploid embryo.

5. A tissue, body fluid, cell, or debris or extract thereof from a non-human animal or its offspring, wherein the non-human animal is prepared by the method according to any one of claims 2 to 4.

6. A non-human animal prepared by the method according to any one of claims 1 to 4.

7. A set of primers, comprising: an upstream primer that has at least 85% or at least 90% or at least 91% or at least 92% or at least 93% or at least 94% or at least 95% or at least 96% or at least 97% or at least 98% or at least 99%, or 100% identity to a sequence as shown by SEQ ID NO: 19; and a downstream primer that has at least 85% or at least 90% or at least 91% or at least 92% or at least 93% or at least 94% or at least 95% or at least 96% or at least 97% or at least 98% or at least 99%, or 100% identity to a sequence as shown by SEQ ID NO: 20.

8. The set of primers according to claim 7, further comprising: an upstream primer that has at least 85% or at least 90% or at least 91% or at least 92% or at least 93% or at least 94% or at least 95% or at least 96% or at least 97% or at least 98% or at least 99%, or 100% identity to a sequence as shown by SEQ ID NO: 21; and a downstream primer that has at least 85% or at least 90% or at least 91% or at least 92% or at least 93% or at least 94% or at least 95% or at least 96% or at least 97% or at least 98% or at least 99%, or 100% identity to the sequence as shown by SEQ ID NO: 22;
preferably, the set of primers further comprises: an upstream primer that has at least 85% or at least 90% or at least 91% or at least 92% or at least 93% or at least 94% or at least 95% or at least 96% or at least 97% or at least 98% or at least 99%, or 100% identity to the sequence as shown by SEQ ID NO: 23; and a downstream primer that has at least 85% or at least 90% or at least 91% or at least 92% or at least 93% or at least 94% or at least 95% or at least 96% or at least 97% or at least 98% or at least 99%, or 100% identity to the sequence as shown by SEQ ID NO: 24;
more preferably, the set of primers further comprises: an upstream primer that has at least 85% or at least 90% or at least 91% or at least 92% or at least 93% or at least 94% or at least 95% or at least 96% or at least 97% or at least 98% or at least 99%, or 100% identity to the sequence as shown by SEQ ID NO: 25; and a downstream primer that has at least 85% or at least 90% or at least 91% or at least 92% or at least 93% or at least 94% or at least 95% or at least 96% or at least 97% or at least 98% or at least 99%, or 100% identity to the sequence as shown by SEQ ID NO: 26.

9. Use of the set of primers according to claim 7 or 8 in preparation of a cell strain, a cell line or a non-human animal;
preferably, the animal is a mammal;
preferably, the animal is selected from a group consisting of pig, rat, mouse, hamster, rabbit, pig, bovine, deer, sheep, goat, chicken, cat, horse, dog, orangutan, and monkey;
preferably, the mammal is a rodent animal;
preferably, the animal is a murine;
preferably, the animal is selected from an adult murine;
preferably, the animal is selected from a fetal murine;
preferably, the animal is an animal with a humanized gene;
preferably, the gene is ACE2.

10. A targeting vector, comprising a 5' homology arm, a fragment of human ACE2 gene, and an SV40 polyA that are linked;
preferably, the 5' homology arm is homologous to a 5' target sequence of a target locus in a genome;
preferably, the 5' homology arm, the fragment of human ACE2 gene and the SV40 polyA sequence are linked and inserted into the targeting vector using the set of primers according to claim 7 or 8;
preferably, the targeting vector is used for inserting a CDS region of human ACE2 gene downstream of a promoter and 5' UTR region of an animal gene, so as to initiate expression of the target human gene using the promoter of the animal gene;
preferably, the 5' homology arm comprises a sequence having at least 85% or at least 90% or at least 91% or at least 92% or at least 93% or at least 94% or at least 95% or at least 96% or at least 97% or at least 98% or at least 99%, or 100% identity to the sequence as shown by SEQ ID NO: 15;
preferably, the CDS region of ACE2 gene comprises a sequence having at least 85% or at least 90% or at least 91% or at least 92% or at least 93% or at least 94% or at least 95% or at least 96% or at least 97% or at least 98% or at least 99%, or 100% identity to the sequence as shown by SEQ ID NO: 12;
preferably, the SV40 polyA comprises a sequence having at least 85% or at least 90% or at least 91% or at least 92% or at least 93% or at least 94% or at least 95% or at least 96% or at least 97% or at least 98% or at least 99%, or 100% identity to the sequence as shown by SEQ ID NO: 14;
preferably, the SV40 polyA is located downstream of the CDS region;
preferably, the targeting vector further comprises a 3' homology arm which is homologous to a 3' target sequence of the target locus in the genome;
preferably, the 3' homology arm comprises a sequence having at least 85% or at least 90% or at least 91% or at least 92% or at least 93% or at least 94% or at least 95% or at least 96% or at least 97% or at least 98% or at least 99%, or 100% identity to the sequence as shown by SEQ ID NO: 16;
preferably, the targeting vector further comprises a selection marker PGK-Puro that has a sequence having at least 85% or at least 90% or at least 91% or at least 92% or at least 93% or at least 94% or at least 95% or at least 96% or at least 97% or at least 98% or at least 99%, or 100% identity to the sequence as shown by SEQ ID NO: 17;
preferably, the targeting vector further comprises a Frt site that has a sequence having at least 85% or at least 90% or at least 91% or at least 92% or at least 93% or at least 94% or at least 95% or at least 96% or at least 97% or at least 98% or at least 99%, or 100% identity to the sequence as shown by SEQ ID NO: 18;
preferably, the targeting vector comprises, linked in turn: the 5' homology arm, the fragment of human ACE2 gene, the SV40 poly A, the Frt site, the PGK-Puro, the Frt site, and the 3' homology arm;
preferably, the animal is a mammal;
preferably, the animal is selected from a group consisting of pig, rat, mouse, hamster, rabbit, pig, bovine, deer, sheep, goat, chicken, cat, horse, dog, orangutan, and monkey;
preferably, the mammal is a rodent animal;
preferably, the animal is a murine;
preferably, the animal is selected from an adult murine;
preferably, the animal is selected from a fetal murine;
preferably, the animal is an animal with a humanized gene;
preferably, the gene is ACE2.

11. Use of the targeting vector according to claim 10 in preparation of a non-human animal,
preferably, the animal is a mammal;
preferably, the animal is selected from a group consisting of pig, rat, mouse, hamster, rabbit, pig, bovine, deer, sheep, goat, chicken, cat, horse, dog, orangutan, and monkey;
preferably, the mammal is a rodent animal;
preferably, the animal is a murine;
preferably, the animal is selected from an adult murine;
preferably, the animal is selected from a fetal murine;
preferably, the animal is an animal with a humanized gene;
preferably, the gene is ACE2.

12. A method for preparing the targeting vector according to claim 10, comprising the following step:
performing an overlap PCR reaction using PCR amplification products of the 5' homology arm, the CDS region of human ACE2 gene and the SV40 polyA, to obtain a continuous fragment 5arm-hACE-SV40;
wherein, a system of the overlap PCR reaction comprises:
about 25 µL of 2×Phanta Max Buffer;
about 1 µL of dNTP Mix;
about 2 µL of 10 µM upstream primer;
about 2 µL of 10 µM downstream primer;
about 1 µL of DNA Polymerase; and
H₂O, making up to about 50 µL;
about 20-200 ng of each of the PCR amplification products of the 5' homology arm, the CDS region of human ACE2 gene, and SV40 polyA as templates;
preferably, the overlap PCR reaction comprises the steps of starting the PCR reaction at about 62-68°C, and decreasing by about 0.2-0.8°C each cycle;
preferably, a set of primers for amplifying the 5' homology arm comprises an upstream primer that has a sequence having at least 85% or at least 90% or at least 91% or at least 92% or at least 93% or at least 94% or at least 95% or at least 96% or at least 97% or at least 98% or at least 99%, or 100% identity to the sequence as shown by SEQ ID NO: 19 and a downstream primer that has a sequence having at least 85% or at least 90% or at least 91% or at least 92% or at least 93% or at least 94% or at least 95% or at least 96% or at least 97% or at least 98% or at least 99%, or 100% identity to the sequence as shown by SEQ ID NO: 20;
preferably, a set of primers for amplifying the CDS region of human ACE2 gene comprises an upstream primer that has a sequence having at least 85% or at least 90% or at least 91% or at least 92% or at least 93% or at least 94% or at least 95% or at least 96% or at least 97% or at least 98% or at least 99%, or 100% identity to the sequence as shown by SEQ ID NO: 21 and a downstream primer that has a sequence having at least 85% or at least 90% or at least 91% or at least 92% or at least 93% or at least 94% or at least 95% or at least 96% or at least 97% or at least 98% or at least 99%, or 100% identity to the sequence as shown by SEQ ID NO: 22;
preferably, a set of primers for amplifying the SV40 polyA comprises an upstream primer that has a sequence having at least 85% or at least 90% or at least 91% or at least 92% or at least 93% or at least 94% or at least 95% or at least 96% or at least 97% or at least 98% or at least 99%, or 100% identity to the sequence as shown by SEQ ID NO: 23 and a downstream primer that a sequence having has at least 85% or at least 90% or at least 91% or at least 92% or at least 93% or at least 94% or at least 95% or at least 96% or at least 97% or at least 98% or at least 99%, or 100% identity to the sequence as shown by SEQ ID NO: 24;
preferably, the method further comprises the following steps:
subjecting the fragment 5arm-hACE-SV40 to AgeI+MluI double digestion, subjecting the 3' homology arm to AscI+HindIII double digestion, and ligating the digested fragments respectively to obtain the targeting vector;
preferably, a set of primers for the 3' homology arm fragment comprises an upstream primer that has a sequence having at least 85% or at least 90% or at least 91% or at least 92% or at least 93% or at least 94% or at least 95% or at least 96% or at least 97% or at least 98% or at least 99%, or 100% identity to the sequence as shown by SEQ ID NO: 25 and a downstream primer that has a sequence having at least 85% or at least 90% or at least 91% or at least 92% or at least 93% or at least 94% or at least 95% or at least 96% or at least 97% or at least 98% or at least 99%, or 100% identity to the sequence as shown by SEQ ID NO: 26.

13. A method for preparing a cell strain or a cell line, wherein the method uses the set of primers according to claim 7 or 8;
preferably, the method comprises a step of using the targeting vector according to claim 10;
preferably, the method comprises a step of introducing a target human-derived gene into an animal cell, so that the animal cell expresses a CDS of the target human-derived gene;
preferably, the target gene is ACE2;
preferably, the animal is a mammal;
preferably, the animal is selected from a group consisting of pig, rat, mouse, hamster, rabbit, pig, bovine, deer, sheep, goat, chicken, cat, horse, dog, orangutan, and monkey;
preferably, the animal is a rodent animal;
preferably, the animal is a murine;
preferably, the animal is selected from an adult murine; preferably, the animal is a murine;
preferably, the animal is selected from a fetal murine;
preferably, the animal is an animal with a humanized gene;
preferably, the cell is an embryonic stem cell;
preferably, the method comprises the following steps:
(1) preparing the targeting vector according to claim 10,
(2) introducing the targeting vector and a vector linked with an sgRNA into an animal-derived embryonic stem cell, and
(3) culturing the embryonic stem cell obtained in step (2) into a clone, to obtain
the cell strain or cell line;
preferably, the sgRNA is selected from a group of sgRNAs that has a sequence having at least 85% or at least 90% or at least 91% or at least 92% or at least 93% or at least 94% or at least 95% or at least 96% or at least 97% or at least 98% or at least 99%, or 100% identity to the sequence as shown by SEQ ID NO: 1 or SEQ ID NO: 2;
or preferably, the animal is a mammal;
preferably, the animal is selected from a group consisting of pig, rat, mouse, hamster, rabbit, pig, bovine, deer, sheep, goat, chicken, cat, horse, dog, orangutan, and monkey;
more preferably, the mammal is a rodent animal;
more preferably, the rodent is a murine;
preferably, the animal is selected from an adult murine;
preferably, the animal is selected from a fetal murine;
preferably, the animal is an animal with a humanized gene;
preferably, the gene is ACE2.

14. A cell strain or a cell line prepared by the method according to claim 13.

15. A method for preparing a non-human animal, comprising a step of using the set of primers according to claim 7 or 8;
preferably, the method comprises a step of using the targeting vector according to claim 10;
preferably, the method comprises a step of injecting the cell according to claim 14 into an animal;
preferably, the animal is a mammal;
preferably, the animal is selected from a group consisting of pig, rat, mouse, hamster, rabbit, pig, bovine, deer, sheep, goat, chicken, cat, horse, dog, orangutan, and monkey;
more preferably, the mammal is a rodent animal;
more preferably, the rodent is a murine;
preferably, the animal is selected from an adult murine;
preferably, the animal is selected from a fetal murines;
preferably, the animal is an animal with a humanized gene;
preferably, the gene is ACE2.

16. A tissue, a body fluid, a cell, and debris or extracts thereof from a non-human animal or its offspring, wherein the non-human animal is prepared by the method according to claim 15.

17. Use of a humanized animal model or its offspring obtained by the method according to claim 15 in preparation of a human antibody, or as a model for pharmacological, immunological, microbiological and medical research, or for etiology research and/or for development of a new diagnostic and/or therapeutic strategy by preparing and using a laboratory animal disease model, or for screening, verification, evaluation or study of ACE2 gene function, an ACE2 antibody, a medicament targeting ACE2 and efficacy thereof.
